# EUROPÄISCHE PATENTSCHRIFT

(11) **EP 1 604 616 B1**
(45) Veröffentlichungstag und Bekanntmachung des Hinweises auf die Patenterteilung: **24.08.2016**
(21) Anmeldenummer: 05019175.8
(22) Anmeldetag: 26.10.2000
(51) Int. Cl.: A61B 17/68

(54) **Chirurgisches Verbindungselement zur Fixierung benachbart angeordneter Knochenplatten**
Surgical instrument for fixation of adjacent bone plates
Élement de connexion pour la fixation des plaques osseuses avoisinantes

(30) Priorität: 30.10.1999 DE 19952359
(43) Veröffentlichungstag der Anmeldung: 14.12.2005
(62) Teilanmeldung aus: 00969554.5
(73) Patentinhaber: Aesculap AG, 78532 Tuttlingen (DE)
(72) Erfinder: Lerch, Karl-Dieter Dr., 58452 Witten (DE); Fischer, Manfred, 73479 Ellwangen (DE); Steinhilper, Klaus-Dieter, 78532 Tuttlingen (DE); Weisshaupt, Dieter, 78194 Immendingen (DE)
(74) Vertreter: Hoeger, Stellrecht & Partner Patentanwälte mbB

(56) Entgegenhaltungen:
- EP-A- 0 787 466
- US-A- 5 814 071
- US-A- 5 921 986
- PATENT ABSTRACTS OF JAPAN Bd. 017, Nr. 670 (C-1139), 9. Dezember 1993 (1993-12-09) -& JP 05 220174 A (YUMIKO SHIMA), 31. August 1993 (1993-08-31)

## Beschreibung

Die Erfindung betrifft ein chirurgisches Verbindungselement zur Fixierung benachbart angeordneter Knochenplatten, umfassend ein erstes Anlageelement, ein zweites Anlageelement und ein Kopplungselement, mittels welchem erstes und zweites Anlageelement derart miteinander koppelbar sind, daß zwischen dem ersten und zweiten Anlageelement liegende Knochenplatten fixierbar sind, wobei das Kopplungselement mindestens eine Fixierungsausnehmung aufweist, in die ein entsprechendes Halteelement des zweiten Anlageelements zur Kopplung dieses zweiten Anlageelements an das Kopplungselement eingreifbar ist.

Um dem Operateur einen Zugang zum Operationsbereich zu eröffnen, wird beispielsweise bei craniochirurgischen Eingriffen die Schädelkapsel des Patienten dadurch geöffnet, daß mittels Craniotomschnitten unter Ausbildung eines Schnittspalts ein Kalottensegment der Schädelkapsel entnommen wird, um so einen Zugang zum darunter liegenden Gehirn zu erhalten. Im Anschluß an die Operation muß das der Schädelkapsel entnommene Kalottensegment wieder in die Schädelkapsel eingefügt und an der verbliebenen Schädelkalotte fixiert werden.

Aus der DE 296 14 921 U1 ist ein chirurgisches Verbindungselement zur Fixierung eines einer Schädelkapsel entnommenen Kalottensegments an der verbliebenen Schädelkalotte bekannt, bei welchem das Verbindungselement einen in einen Schnittspalt zwischen Kalottensegment und verbliebener Schädelkalotte einführbaren Verbindungsschaft, einen Verbindungskopf zum Überdecken des Schnittspaltes sowie ein vom Verbindungsschaft seitlich vorspringendes, am Kalottensegment sowie der verbliebenen Schädelkalotte anlegbares Anlageelement umfaßt.

Aus der EP 0 787 466 ist eine Vorrichtung zum Fixieren eines aus der Schädelkapsel zum Zwecke eines operativen Eingriffs herausgetrennten Knochenstücks in einem am verbliebenen Schädelbein freigelegten Bereich bekannt, wobei eine mittig gelochte Scheibe als erstes und zweites Anlageelement auf einen Schaft als Kopplungselement aufschiebbar sind und eine Scheibe ausgehende Radialschnitte aufweist. Zum Fixieren der Scheibe an dem Schaft wird ein Werkzeug verwendet. Auch eine Gewindeverbindung kann zum Einsatz kommen.

Aus der US 4,802,477 ist eine Vorrichtung zum Sichern eines Sternums in einer geschlossenen Stellung nach Öffnung bekannt. Ein entsprechender Fixierungskragen greift in eine Gewindestange ein.

Aus der WO 00/49949 A1 ist ein Befestigungssystem bekannt, welches ein Befestigungselement aus einem biokompatiblen Kunststoffmaterial umfaßt, wobei ein im wesentlichen scheibenförmiger Kopf und ein Schaft vorgesehen sind. Der Schaft hat eine im wesentlichen flache erste Oberfläche und eine im wesentlichen flache zweite Oberfläche, wobei diese Oberflächen parallel zueinander sind. Es ist ferner ein im wesentlichen scheibenförmiges Fixierungselement aus einem biokompatiblen starren Kunststoff material vorgesehen.

Dieses hat eine Durchgangsöffnung und Rastzähne, welche mindestens einen Teil der Öffnung definieren. Diese Rastzähne operieren mit den Rastzähnen des Schafts; der Schaft kann durch die Öffnung geführt werden, so daß der Kopf und das Fixierungselement aufeinander zu gedrückt werden können, aber nicht voneinander weg bewegt werden können.

Es ist Aufgabe der Erfindung, ein gattungsgemäßes chirurgisches Verbindungselement derart auszubilden, daß es einfach und auf sichere Weise einsetzbar und auf kostengünstige Weise herstellbar ist und insbesondere körperverträgliche und resorbierbare Materialien verwendbar sind.

Diese Aufgabe wird bei einem chirurgischen Verbindungselement der eingangs beschriebenen Art erfindungsgemäß dadurch gelöst, daß ein Halteelement eine Haltelasche ist und daß das erste Anlageelement und/oder zweite Anlageelement einen hochgezogenen Randbereich zur Erzeugung einer Zugspannung in einer Klemmrichtung aufweist, wobei der hochgezogene Randbereich einen Winkel zu einer Anlagefläche des entsprechenden Anlageelements aufweist, elastisch verformbar ist, um den Winkel zwischen dem Randbereich und der Anlagefläche zu verringern, und dadurch in Klemmstellung eine Kraft ausübt, welche die Klemmung verbessert.

Durch eine derartige Ausgestaltung muß keine Gewindeverbindung oder Klemmverbindung über den ganzen Umfang eines Schafts mehr vorgesehen werden, sondern die Kopplung zwischen dem zweiten Anlageelement und dem Kopplungselement läßt sich auf einfache und doch sichere Weise ausbilden. Dadurch hat man auch eine größere Auswahl an Materialien, die zur Herstellung des chirurgischen Verbindungselements einsetzbar sind, da kein Gewinde mehr geschnitten werden muß.

Bei einem Gewindeschnitt oder auch bei einer Verklemmung über den ganzen Umfang sind bevorzugterweise Metalle zu verwenden, um eine gute Fixierung zu erhalten. Durch die erfindungsgemäße Kopplung, die davon abgeht, insbesondere einen Gewindeschnitt vorsehen zu müssen, lassen sich dann auch beispielsweise weichere Kunststoffmaterialien einsetzen, die eine hohe Körperverträglichkeit oder Resorptionsfähigkeit aufweisen und mit denen sich durch den erfindungsgemäßen Fixierungs- und Kopplungsmechanismus sich eine gute und sichere Verbindung herstellen läßt.

Durch die erfindungsgemäße Ausgestaltung läßt sich insbesondere eine kraftschlüssige Verbindung zwischen dem zweiten Anlageelement und dem Kopplungselement herstellen.

Wenn das erste und/oder zweite Anlageelement einen hochgezogenen Randbereich zur Erzeugung einer Zugspannung in Klemmrichtung aufweist, läßt sich dieser bei der Herstellung der Fixierung, bei welcher Knochenplatten zwischen dem ersten und dem zweiten Anlageelement eingeklemmt werden, verformen. Dadurch wird in der Fixierungsstellung (der Klemmstellung) eine Kraft durch den verformten hochgezogenen Randbereich ausgeübt, der die Verklemmung verbessert und damit eine hohe Fixierungssicherheit bewirkt.

Besonders vorteilhaft ist es, wenn eine Fixierungsstellung des ersten und des zweiten Anlageelements durch Relativbewegung zwischen dem Kopplungselement und dem zweiten Anlageelement quer zur Klemmrichtung verriegelbar ist. Dadurch läßt sich auf einfache Weise mit kostengünstig herstellbaren Verbindungselementen eine sichere Fixierung erreichen.

Aus demselben Grund ist es besonders günstig, wenn eine Fixierungsstellung des ersten und des zweiten Anlageelements durch Relativbewegung zwischen dem Kopplungselement und dem zweiten Anlageelement quer zur Klemmrichtung entriegelbar ist. Beispielsweise läßt sich dann eine Nachjustierung auf einfache Weise während einer Operation durchführen.

Um die Verklemmung von zwischen dem ersten und dem zweiten Anlageelement eingeklemmten Knochenplatten zu verbessern, ist es besonders günstig, wenn das erste und/oder zweite Anlageelement einen Haltekörper aufweisen, welcher in einen Zwischenraum zwischen benachbart angeordneten Knochenplatten einsetzbar ist. Ein solcher Haltekörper dient als zusätzliche Verkeilung und insbesondere bewirkt er auch eine zusätzliche Fixierung in einer Richtung quer zur Klemmrichtung.

Ganz besonders vorteilhaft ist es, wenn ein Haltekörper in Querrichtung zur Klemmrichtung elastisch ausgebildet ist und insbesondere federnd ausgebildet ist. Dadurch läßt sich in dieser Querrichtung der Abstand der Knochenplatten, welche an dem Haltekörper anliegen, in bestimmten Grenzen variieren und insbesondere läßt sich bei der Verwendung von mehreren Verbindungselementen eine Verspannung einer Knochenplatte gegenüber umgebenden Knochenplatten in Querrichtung erreichen.

Da die Verbindungselemente im Körper verbleiben, ist es vorteilhaft, wenn ein solches Verbindungselement aus einem körperverträglichen Material hergestellt ist. Ganz besonders vorteilhaft ist es, wenn das Verbindungselement aus einem resorbierbaren Kunststoffmaterial gefertigt ist. Schnittspalte zwischen den Knochenplatten wachsen nach der Operation wieder zu und bei resorbierbarem Material kann Gewebe auch über das Verbindungselement wachsen, so daß dieses kein "Störfaktor" im Körper ist.

Bei einer vorteilhaften Variante einer Ausführungsform ist das Kopplungselement mit einer Sollbruchstelle versehen. Dadurch kann auf einfache Weise, insbesondere nach erfolgter Fixierung, das Kopplungselement entsprechend verkürzt werden. Ganz besonders vorteilhaft dabei ist es, wenn die Sollbruchstelle so am Kopplungselement angeordnet und so ausgebildet ist, daß ein Bruch im wesentlichen bei einer solchen Zugspannung erzeugbar ist, welche eine optimale Vorspannung der Knochenplatten bewirkt. Dadurch läßt sich die Fixierung durch den Operateur auf einfache Weise erreichen.

Bei einer Variante einer Ausführungsform ist es vorgesehen, daß das erste Anlageelement einstückig oder formschlüssig mit dem Kopplungselement verbunden ist. Das erste Anlageelement muß dann nicht mehr mit dem Kopplungselement vor Verwendung eines Verbindungselements verbunden werden.

Vorteilhafterweise weist das zweite Anlageelement mindestens eine Durchgangsausnehmung für das Kopplungselement auf, so daß das zweite Anlageelement und das Kopplungselement relativ zueinander parallel zur Klemmrichtung beweglich sind.

Schließlich ist es bei einer weiteren Ausführungsform vorgesehen, daß das zweite Anlageelement mit dem Kopplungselement durch eine Relativbewegung eines am zweiten Anlageelement sitzenden Halteteils zum Kopplungselement mit diesem verbindbar ist.

Bei einer Variante einer Ausführungsform ist es vorgesehen, daß das Kopplungselement ein starrer Körper ist. Dies bedeutet, daß das Kopplungselement eine definierte Gestalt hat. Das Kopplungselement kann aber weiterhin elastische Eigenschaften haben, d. h. sich beispielsweise elastisch verformen lassen.

Günstigerweise ist das Kopplungselement so dimensioniert, daß es in einer Querrichtung zu seiner Längsrichtung eine größere Breite aufweist als in der Querrichtung senkrecht dazu. Dadurch ist das Kopplungselement bandförmig ausgebildet. Die längere Seite liegt bei der Positionierung des Verbindungselements in einem Schnittspalt und der Abstand zwischen benachbarten Knochenplatten ist (bei Anliegen an dem Kopplungselement) im wesentlichen durch die Breite der schmaleren Seite bestimmt. Dadurch ist zum einen die Breite des Knochenspaltes verringert und zum anderen läßt sich eine sichere Kopplung zwischen dem zweiten Anlageelement und dem Kopplungselement erreichen, ohne daß beispielsweise ein Gewinde am Kopplungsteil und zweiten Anlageelement vorgesehen werden muß.

Günstigerweise greift das Halteelement des zweiten Anlageelements quer zur Klemmrichtung in eine Fixierungsausnehmung ein.

Ganz besonders günstig ist es, wenn das Kopplungselement eine Mehrzahl von Fixierungsausnehmungen umfaßt, welche in Längsrichtung des Kopplungselements angeordnet sind. Dadurch lassen sich entsprechende Abstände zwischen dem ersten und dem zweiten Anlageelement einstellen, und insbesondere läßt sich der Abstand einstellen, der zur Erzielung einer guten Klemmwirkung notwendig ist.

Günstigerweise sind dabei benachbarte Fixierungsausnehmungen im gleichen Abstand zueinander angeordnet.

Es kann insbesondere vorgesehen sein, daß das Kopplungselement mit einer aus Fixierungsausnehmungen gebildeten Eingriffsleiste versehen ist. Diese ist vorteilhafterweise als Rastleiste ausgebildet, um so auf einfache Weise eine Kopplung und Fixierung zu ermöglichen.

Bei einer Variante einer Ausführungsform ist es vorgesehen, daß das Kopplungselement mindestens zwei Eingriffsleisten aufweist. Günstigerweise sind dabei mindestens zwei Eingriffsleisten bezüglich des Abstands von Fixierungsausnehmungen relativ zum ersten Anlageelement versetzt zueinander angeordnet. Durch die Versetzung läßt sich eine feinere Stufenunterteilung erreichen, ohne daß eine Fixierungsausnehmung und ein zwischen Fixierungsausnehmungen liegender Steg selber geschmälert werden muß.

Bei einer Variante einer Ausführungsform ist eine Seitenfläche des Kopplungselements mit einer Eingriffsleiste versehen. Dadurch stößt die Eingriffsleiste selber nicht an die Knochenplatten an.

Bei einer anderen Variante einer Ausführungsform ist eine Eingriffsleiste zwischen Seitenflächen des Kopplungselements am Kopplungselement angeordnet. Die Eingriffsleiste läßt sich dadurch über einen größeren Flächenbereich anordnen und entsprechend die Kopplung verbessern. Beispielsweise ist es bei einer Anordnung an der Seitenfläche sehr vorteilhaft, wenn gegenüberliegende Seitenflächen jeweils mit einer Eingriffsleiste versehen sind, um die Kopplung durch Erhöhung der Kopplungsfläche zu verbessern. Bei einer Anordnung zwischen den Seitenflächen genügt eine Eingriffsleiste, da eine größere Eingrifffläche bereitstellbar ist.

Es kann vorgesehen sein, daß eine Eingriffsleiste stufenförmig ausgebildet ist. Bei einer weiteren Variante kann es vorgesehen sein, daß eine Eingriffsleiste sägezahnförmig ausgebildet ist. Fertigungstechnisch besonders günstig ist es, wenn eine Eingriffsleiste rechteckförmige Ausnehmungen als Fixierungsausnehmungen umfaßt.

Bei einer vorteilhaften Variante einer Ausführungsform weist eine Fixierungsausnehmung einen sich verjüngenden Querschnitt auf. Bei entsprechender Anpassung des zweiten Anlageelements (d. h. entsprechender Anpassung eines Halteteils davon) läßt sich eine Keilwirkung erzielen zur besseren Fixierung.

Günstig ist es, wenn das zweite Anlageelement eine Haltelasche aufweist, welche in eine Fixierungsausnehmung eintauchbar ist und mittels der sich das zweite Anlageelement mit dem Kopplungselement verbinden läßt.

Bei einer Variante einer Ausführungsform weist eine Haltelasche eine Mehrzahl von Haltezähnen zum gleichzeitigen Eingriff in eine Mehrzahl von Fixierungsausnehmungen auf. Dadurch wird eine erhöhte Fixierungssicherheit erreicht.

Es kann auch vorgesehen sein, daß eine Haltelasche in einem Winkel zu einer Anlagefläche des zweiten Anlageelements angeordnet ist. Dadurch läßt sich beispielsweise eine Fixierungsausnehmung als durchgehende Öffnung mit rechteckigem Querschnitt ausbilden und durch die schräge Anordnung der Haltelasche eine gute Fixierung erreichen.

Ein erfindungsgemäßes Verbindungselement läßt sich auf einfache Weise einsetzen und kostengünstig herstellen, wenn das zweite Anlageelement kraftschlüssig an dem Kopplungselement fixierbar ist.

Die nachfolgende Beschreibung bevorzugter Ausführungsformen der Erfindung dient im Zusammenhang mit der Zeichnung der näheren Erläuterung. Es zeigen:
- Fig. 1: eine perspektivische Ansicht eines einer Schädelkapsel entnommenen Kalottensegments, welches wieder in die verbliebene Schädelkalotte eingesetzt ist, wobei nebeneinander liegende Knochenplatten mit Hilfe von Verbindungselementen gemäß einer ersten Ausführungsform, welche nicht unter die Ansprüche fällt, fixiert sind;
- Fig. 2: eine perspektivische Ansicht einer zweiten Ausführungsform eines Verbindungselements, welches nicht unter die Ansprüche fällt, mit fixierten Knochenplatten;
- Fig. 3: eine perspektivische Ansicht einer dritten Ausführungsform eines Verbindungselements, welches nicht unter die Ansprüche fällt, mit fixierten Knochenplatten;
- Fig. 4: eine perspektivische Ansicht einer vierten Ausführungsform eines Verbindungselements;
- Fig. 5: eine perspektivische Ansicht einer fünften Ausführungsform eines Verbindungselements, welches nicht unter die Ansprüche fällt;
- Fig. 6: eine perspektivische Ansicht einer sechsten Ausführungsform eines Verbindungselements, welches nicht unter die Ansprüche fällt;
- Fig. 7: eine perspektivische Ansicht einer siebten Ausführungsform eines Verbindungselements, welches nicht unter die Ansprüche fällt;
- Fig. 8: eine perspektivische Ansicht einer achten Ausführungsform eines Verbindungselements;
- Fig. 9: eine perspektivische Ansicht einer neunten Ausführungsform eines Verbindungselements;
- Fig. 10: eine perspektivische Ansicht einer zehnten Ausführungsform eines Verbindungselements;
- Fig. 11: eine perspektivische Ansicht einer elften Ausführungsform eines Verbindungselements;
- Fig. 12: eine perspektivische Ansicht einer zwölften Ausführungsform eines Verbindungselements, welche nicht unter die Ansprüche fällt;
- Fig. 13: eine perspektivische Ansicht einer 13. Ausführungsform eines Verbindungselements;
- Fig. 14: eine perspektivische Ansicht einer 14. Ausführungsform eines Verbindungselements, welches nicht unter die Ansprüche fällt;
- Fig. 15: eine perspektivische Ansicht einer 15. Ausführungsform eines Verbindungselements;
- Fig. 16: eine perspektivische Ansicht einer 16. Ausführungsform eines Verbindungselements und
- Fig. 17: eine perspektivische Ansicht einer 17. Ausführungsform eines Verbindungselements, welches nicht unter die Ansprüche fällt.

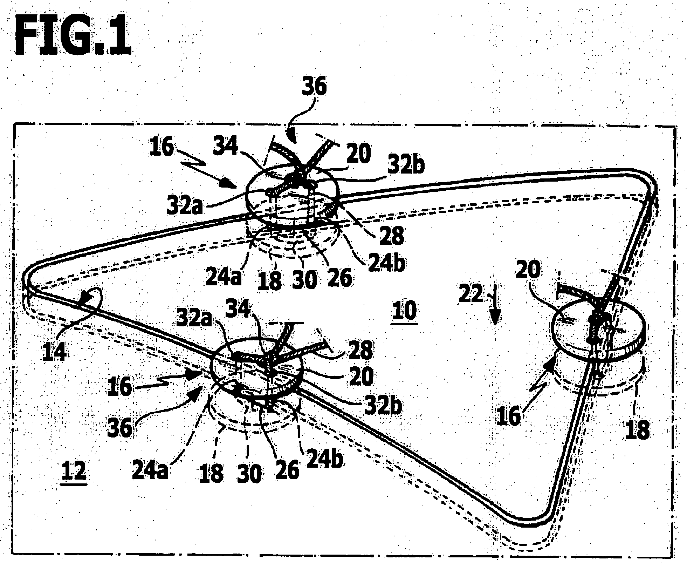

Ein Anwendungsbeispiel für ein erfindungsgemäßes Verbindungselement ist die Fixierung eines Kalottensegments 10, wie in Fig. 1 gezeigt, an einer umgebenden verbliebenen Schädelkalotte 12. Das Kalottensegment 10 wurde durch Craniotomschnitte entfernt, welche einen Schnittspalt 14 gebildet haben, um einem Operateur einen Zugang zum unter dem Kalottensegment 10 liegenden Gehirn zu verschaffen. Nach erfolgter Operation wird dieses Kalottensegment 10 wieder eingesetzt. Mit Hilfe von Verbindungselementen 16, beispielsweise mittels dreier solcher Verbindungselemente 16, wie in Fig. 1 dargestellt, werden die benachbarten Knochenplatten 10 und 12 fixiert, so daß das Kalottensegment 10 wieder mit der Schädelkalotte 12 verwachsen kann. Dazu ist ein solches Verbindungselement 16 aus einem körperverträglichen Material gefertigt und insbesondere aus einem resorbierbaren Material, so daß Körpergewebe mit dem Verbindungselement verwachsen kann.

Bei einer Ausführungsform eines Verbindungselements, welche nicht unter die Ansprüche fällt und welche in Fig. 1 gezeigt ist, umfaßt ein solches Verbindungselement 16 ein erstes scheibenförmiges Anlageelement 18 und ein zweites Anlageelement 20, welches grundsätzlich gleich aufgebaut ist wie das erste Anlageelement. Das erste Anlageelement 18 wird unter das Kalottensegment 10 und die Schädelkalotte 12 gelegt und das zweite Anlageelement 20 in einem Abstand dazu über das Kalottensegment 10 und die Schädelkalotte 12. Durch Fixierung des ersten Anlageelements 18 relativ zum zweiten Anlageelement 20 und Ausübung einer Klemmkraft in eine Klemmrichtung 22, welche bei dem gezeigten Ausführungsbeispiel im wesentlichen senkrecht zu den Knochenplatten 10 und 12 ist, lassen sich dann das benachbarte Kalottensegment 10 und die Schädelkalotte 12 gegeneinander fixieren.

Das erste Anlageelement 18 weist zwei in einem Abstand angeordnete Durchführungsausnehmungen 24a, 24b auf, deren Verbindungslinie 26 bevorzugterweise auf einer Durchmesserlinie liegt. Ein Faden oder Draht 28 als Kopplungselement, welches linear flexibel ist in dem Sinne, daß es bezogen auf einen tangentialen Richtungsvektor senkrecht zu diesem Richtungsvektor keine Vorzugsrichtung bezüglich der Flexibilität gibt, ist so durch die beabstandeten Durchführungsausnehmungen 24a und 24b im ersten Anlageelement 18 geschleift, daß zwischen den beiden Durchführungsausnehmungen 24a und 24b ins Schädelinnere weisend eine Schleife 30 gebildet ist.

Das Kopplungselement 28 ist dann durch den Schnittspalt 14 vom Schädelinneren weg durch entsprechende Ausnehmungen 32a, b im zweiten Anlageelement 20 geführt. Wird eine Zugkraft nach außen (entgegen der Klemmrichtung 22) auf entsprechende Enden des Kopplungselements 28 ausgeübt, dann bewirkt dies, daß die Schleife 30 an dem ersten Anlageelement 18 anliegt.

Bei dem in Fig. 1 gezeigten Ausführungsbeispiel sind das erste Anlageelement 18 und das zweite Anlageelement 20 über einen Knoten 34 gegeneinander fixiert. Dazu werden entsprechende Enden des Kopplungselements 28 nach Durchschleifen durch die Ausnehmungen 32a und 32b des zweiten Anlageelements und Anziehen des Kopplungselements quer zur Klemmrichtung 22 geführt und miteinander verknotet, wobei eine Zugspannung ausgeübt wird, um eine Klemmkraft dadurch auszuüben, daß über die Schleife 30 das zweite Anlageelement 20 nach oben gezogen wird und gegen das Kalottensegment 10 und die Schädelkalotte 12 gespannt ist. Durch den Knoten 34 wird diese Fixierungsstellung oder Klemmstellung gesichert, d. h. die Fixierungsstellung 36 wird verriegelt.

Auf diese Weise sind dann über die Klemmwirkung des ersten Anlageelements 18 und des zweiten Anlageelements 20 auf die zwischenliegenden Knochenplatten das Kalottensegment 10 und die Schädelkalotte 12 zumindest in dem Bereich um ein Verbindungselement relativ zueinander fixiert. Durch die Verwendung mehrerer solcher Verbindungselemente 16 läßt sich dann das Kalottensegment 10 gegenüber der verbliebenen Schädelkalotte 12 im gesamten fixieren.

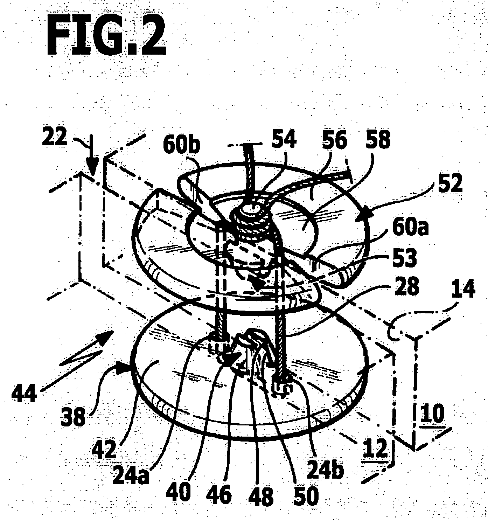

Bei einem weiteren Ausführungsbeispiel, welches in Fig. 2 gezeigt ist und nicht unter die Ansprüche fällt, ist das erste Anlageelement 38 grundsätzlich gleich aufgebaut, wie für das erste Ausführungsbeispiel beschrieben. Jedoch weist das erste Anlageelement bevorzugterweise zentral angeordnet einen Haltekörper 40 auf, welcher sich über eine Anlagefläche 42 erhebt. Das erste Anlageelement 38 wird bei dieser zweiten Ausführungsform 44 eines Verbindungselements mit dem Haltekörper 40 in den Schnittspalt 14 zwischen den benachbart angeordneten Knochenplatten 10 und 12 positioniert, um eine zusätzliche Verkeilung und damit eine bessere Fixierung zwischen den beiden Knochenplatten 10 und 12 zu bewirken.

Bevorzugterweise ist der Haltekörper 40 quer zur Klemmrichtung 22 elastisch und insbesondere federnd ausgebildet, so daß eine gewisse Variabilität bei der Bewegung der beiden Knochenplatten 10 und 12 aufeinander zu oder voneinander weg besteht, um ein optimales Fixierungsergebnis zu erreichen.

Bei dem in Fig. 2 gezeigten Ausführungsbeispiel umfaßt der Haltekörper 40 zwei gegenüberliegende Stiftteile 46 und 48, zwischen welchen ein Zwischenraum 50 gebildet ist. Die Stiftteile 46 und 48 sind dabei nach außen kegelstumpfförmig ausgebildet. Der Zwischenraum 50 bestimmt im wesentlichen, wie weit die Knochenplatten im Schnittspalt aufeinanderzuschiebbar sind; stoßen die beiden Stiftteile 46 und 48 aneinander, dann ist der minimale Abstand zwischen den Knochenplatten erreicht.

Bei der zweiten Ausführungsform 44 ist im Zentrum des zweiten Anlageelements 52 ein Pflockelement 54 angeordnet, welches insbesondere in einer gegenüber einer äußeren Oberfläche 56 gebildeten Vertiefung 58 sitzt, damit dieses Pflockelement 54 nicht oder nur geringfügig über die Oberfläche 56 hinausragt.

In dem zweiten Anlageelement 52 sind in radialer Richtung seitlich offene keilförmige Ausnehmungen 60a, 60b gebildet, durch die das flexible Kopplungselement 28 einführbar ist, um es um das Pflockelement 24 umschleifen und insbesondere umwickeln zu können.

Die Klemmkraft zwischen dem ersten Anlageelement 38 und dem zweiten Anlageelement 52 wird grundsätzlich, wie bereits oben für die erste Ausführungsform beschrieben, erzeugt. Die Fixierungsstellung wird dabei dadurch verriegelt, d. h. fixiert, daß die jeweiligen Enden des linear flexiblen Kopplungselements 28 um das Pflockelement 54 so gewickelt werden, daß die beiden Lageelemente 38 und 52 gegeneinander mit dazwischenliegenden Knochenplatten 10 und 12 gehalten sind. Die Ausnehmungen 60a und 60b dienen dabei als Einführungsschlitze für ein fadenförmiges Kopplungselement 28.

Das zweite Anlageelement 52 kann dabei ebenfalls einen zentral angeordneten Haltekörper 53 aufweisen, der insbesondere gleich wie der Haltekörper 40 des ersten Anlageelements ausgebildet ist.

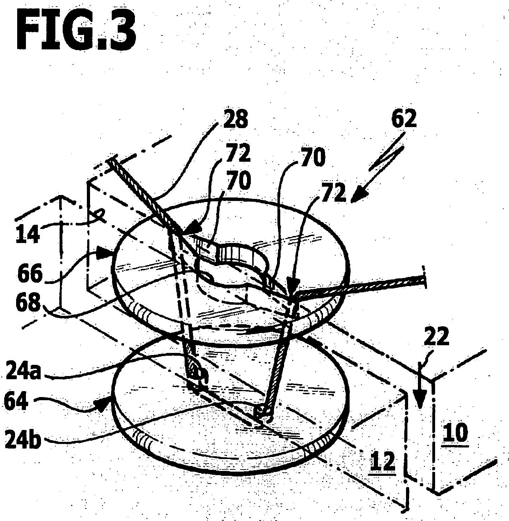

Bei einer dritten Ausführungsform eines Verbindungselements 62, welche in Fig. 3 gezeigt ist und nicht unter die Ansprüche fällt, ist das erste Anlageelement 64 wie das erste Anlageelement 18 gemäß dem erstem Ausführungsbeispiel (Fig. 1) ausgebildet. Das zweite Anlageelement 66 weist eine zentrale Öffnung 68 als Durchführungsausnehmung für das linear flexible Kopplungselement 28 zum Durchführen von diesem auf. Ausgehend von dieser zentralen Öffnung 68 sind gegenüberliegend in radialer Richtung keilförmige Ausnehmungen 70 gebildet. Am radial außen liegenden Ende einer solchen Ausnehmung 70 ist eine Klemmaufnahme 72 als

Fixierungsausnehmung gebildet, in die das flexible Kopplungselement 28 einklemmbar ist, um dieses gegenüber dem zweiten Anlageelement 66 zu fixieren.

Eine Klemmaufnahme 72 ist dabei bevorzugterweise bezogen auf eine Projektion einer Durchführungsausnehmung 24a bzw. 24b des ersten Anlageelements 64 in Klemmrichtung 22 auf das zweite Anlageelement 66 (bei entsprechender Ausrichtung von erstem Anlageelement 64 und zweitem Anlageelement 66) in radialer Richtung weiter außen liegend. Dadurch lassen sich durch Zug quer zur Klemmrichtung 22 Knochenplatten zwischen dem ersten Anlageelement 64 und dem zweiten Anlageelement einklemmen, wobei mittels der Klemmaufnahme 72 eine Fixierungsstellung fixierbar ist.

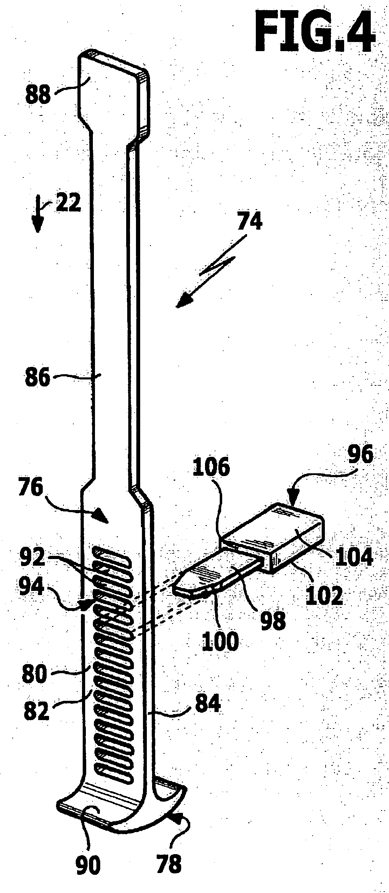

Bei einer vierten Ausführungsform, welche in Fig. 4 gezeigt ist, umfaßt ein erfindungsgemäßes Verbindungselement 74 ein Kopplungselement 76, an dem einstückig ein erstes Anlageelement 78 gebildet ist. Das Kopplungselement 76 mit dem ersten Anlageelement 78 ist dabei starr in dem Sinne, daß es eine feste Gestalt hat.

Das Kopplungselement 76 weist einen unteren Bereich 80 auf, der im Querschnitt rechteckförmig ist, wobei eine Rechteckseite länger ist als eine andere, so daß der untere Bereich 80 bandförmig ausgestaltet ist. Das erste Anlageelement 78 ist dabei quer zur längeren Seite 82 des unteren Bereichs 80, so daß die schmalere Seite 84 den minimalen Abstand benachbarter Knochenplatten bei der Fixierung bestimmt.

An den unteren Bereich 80 schließt sich ein mittlerer Bereich 86 an, auf welchen ein oberer Bereich 88 folgt. Der mittlere Bereich 86 weist einen kleineren Querschnitt bezüglich der längeren Seite 82 als der untere Bereich 80 und der obere Bereich 88 auf, so daß insbesondere durch den Übergang zwischen dem mittleren Bereich 86 und dem oberen Bereich 88 ein Griffelement gebildet ist, mit dessen Hilfe sich das Kopplungselement 76 halten oder bei Bedarf eine Zugspannung auf das erste Anlageelement 78 mit seinen Anlageflächen 90 ausüben läßt.

Der mittlere Bereich 86 ist mit parallel zu den Anlageflächen 90 des ersten Anlageelements 78 versehenen durchgehenden schlitzartigen Ausnehmungen 92 versehen, welche selber zueinander in gleichmäßigem Abstand angeordnet sind, so daß durch die Ausnehmungen 92 eine Eingriffsleiste 94 gebildet ist. Dadurch läßt sich ein zweites Anlageelement 96 in verschiedenen Höhenstellungen mit dem Kopplungselement 76 bezogen auf das erste Anlageelement 78 kraftschlüssig verbinden.

Das zweite Anlageelement 96 weist eine Haltelasche 98 auf, welche an eine Ausnehmung 92 angepaßt ist und mittels weicher das zweite Anlageelement 96 in eine Ausnehmung 92 quer zu der Klemmrichtung 22 eingeschoben wird. Die eben ausgebildete Haltelasche 98 ist dazu an ihrem vorderen Ende mit abgeschrägten Kanten 100 versehen, um das Einführen in eine Ausnehmung 92 zu erleichtern.

Die Haltelasche 98 des zweiten Anlageelements 96 sitzt an einem eine Anlagefläche 102 für eine Knochenplatte bildenden Element 104. Die Haltelasche 98 sitzt dabei an einer Stirnfläche des Elements 104 derart, daß eine Quer-Anlagefläche 106 für das Kopplungselement 76 gebildet ist, mittels welcher sich ein eingeschobenes zweites Anlageelement an dem Kopplungselement 76 quer zur Klemmrichtung 22, welche der Längsrichtung des Kopplungselements 76 entspricht, abstützen kann.

Das Verbindungselement 74 wird mit den Anlageflächen 90 unter zu fixierenden Knochenplatten positioniert, wobei dann der untere Bereich 80 des Kopplungselements 76 sich im Schnittspalt 14 befindet. Zur Fixierung und gleichzeitig Ausübung einer Klemmkraft wird das zweite Anlageelement 96 in die entsprechende Ausnehmung 92 quer zur Klemmrichtung 22 geschoben. In der Ausnehmung 92 ist dann das zweite Anlageelement 96 kraftschlüssig fixiert. Insbesondere ist dabei die Haltelasche 98 derart elastisch ausgebildet, daß sie in Klemmrichtung 22 biegbar ist und entsprechend ein eingeschobenes zweites Anlageelement 96 in Richtung des ersten Anlageelements 78 eine Klemmkraft ausübt.

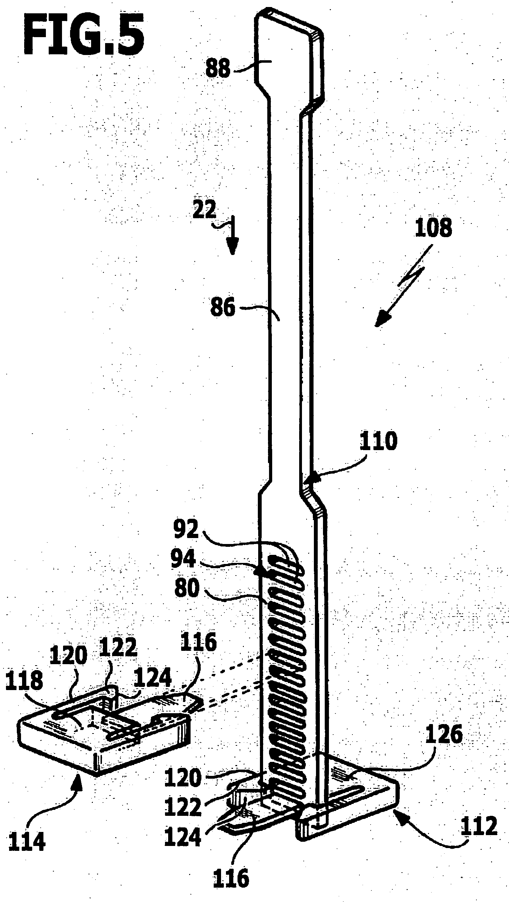

Bei einer fünften Ausführungsform eines Verbindungselements 108, welche in Fig. 5 gezeigt ist und nicht unter die Ansprüche fällt, ist das Kopplungselement 110 grundsätzlich gleich aufgebaut wie bei der eben beschriebenen vierten Ausführungsform. Jedoch ist ein erstes Anlageelement 112 nicht einstückig mit dem Kopplungselement 110 gebildet, sondern als getrenntes Element, welches im wesentlichen gleich wie ein zweites Anlageelement 114 aufgebaut ist. Das zweite Anlageelement 114 umfaßt wiederum eine Haltelasche 116 zum Einschub in eine entsprechende Ausnehmung 92. Die Haltelasche 116 sitzt an einem Element 118, an dem seitlich jeweils Fixierungslaschen 120 in der Form von Haltekrallen sitzen. Bei Einschub des zweiten Anlageelements 114 in eine Ausnehmung 92 des Kopplungselements 110 umgreifen diese Fixierungslaschen 120 das Kopplungselement 110, um eine zusätzliche Verbindung mit diesem zu bewirken und so insgesamt die Fixierung zu verbessern. Dazu weist eine Fixierungslasche 120 an einem Ende einen Haltezahn 122 auf, welcher an seinem vorderen Ende eine abgeschrägte Kante 124 hat, damit der Haltezahn über das Kopplungselement 110 schiebbar ist. Die Fixierungslasche 120 ist dazu derart an dem Element 118 angeordnet, daß sie quer zur Einschubrichtung beweglich ist. Bei eingeschobenem zweiten Anlageelement sitzt das Kopplungselement 110 zwischen dem Element 118 und den Haltezähnen 122.

Das gleich wie das zweite Anlageelement ausgestaltete erste Anlageelement 112 wird an einem unteren Ende des Kopplungselements 110 eingeschoben und entsprechend wie oben für das zweite Halteelement beschrieben an dem Kopplungselement 110 fixiert.

Bevorzugterweise ist eine Anlagefläche 126 des ersten bzw. zweiten Anlageelements 112 bzw. 114 eben, um eine gute Anlage zu ermöglichen.

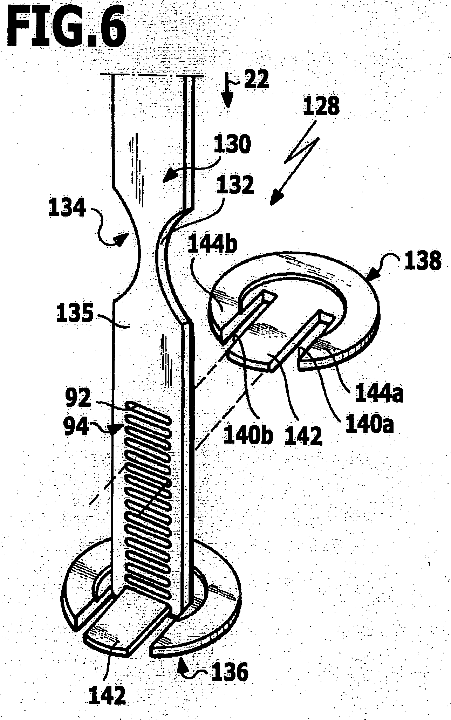

Bei einer sechsten Ausführungsform 128 eines Verbindungselements, welche in Fig. 6 gezeigt ist und nicht unter die Ansprüche fällt, ist das Kopplungselement 130 grundsätzlich wie oben beschrieben aufgebaut. Jedoch weist das Kopplungselement als zusätzliches Merkmal eine Sollbruchstelle 132 auf, die beispielsweise dadurch gebildet ist, daß das Kopplungselement 130 einen Bereich 134 mit verringertem Querschnitt bezüglich einer längeren Seite 135 aufweist. Die

Sollbruchstelle dient dazu, einen Bruch des Kopplungselements 130 zu bewirken, wenn eine Zugspannung erzeugt wird, welche eine optimale Verspannung der Knochenplatten bewirkt.

Bei dem in Fig. 6 gezeigten Ausführungsbeispiel sind wiederum das erste Anlageelement 136 und das zweite Anlageelement 138 gleich ausgebildet. Ein solches Anlageelement 136 bzw. 138 ist scheibenförmig mit schlitzartigen Ausnehmungen 140a, 140b, welche parallel zueinander beabstandet vom Scheibenrand nach innen eines solchen Anlageelements 136 bzw. 138 verlaufen. Dadurch ist zwischen den Ausnehmungen 140a und 140b eine Haltelasche 142 zum Einschieben in eine Ausnehmung 92 des Kopplungselements 130 gebildet. An den Ausnehmungen 140a, b anliegende Seitenflanken 144a, b bewirken einen zusätzlichen Halt eines eingeschobenen Anlageelements an dem Kopplungselement 130.

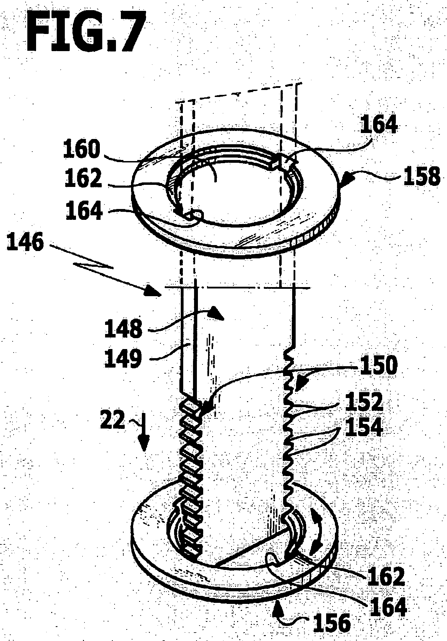

Bei einer siebten Ausführungsform 146, welche in Fig. 7 gezeigt ist, ist ein Kopplungselement 148 an seinen gegenüberliegenden Seitenflächen (an der schmalen Seite 149) jeweils mit Eingriffsleisten 150 versehen. Eine Eingriffsleiste 150 ist als Zahnleiste mit Zähnen 152 und zwischen den Zähnen liegenden Fixierungsausnehmungen 154 ausgebildet.

Das erste Anlageelement 156 und das zweite Anlageelement 158 sind gleich aufgebaut mit einer zentralen Durchführungsöffnung 160, durch welche beispielsweise das zweite Anlageelement 158 auf das Kopplungselement 148 schiebbar ist. In der Durchführungsöffnung 160 sitzt eine umlaufende Haltelasche 162, welche zum Eingriff in eine Fixierungsausnehmung 154 eine Eingriffsleiste 150 dient. Die Haltelasche 162 ist durch radial gegenüberliegende Ausnehmungen 164 durchbrochen, deren Breite und Abstand voneinander an die entsprechenden Dimensionierungen des Kopplungselements 148 angepaßt sind, um über die Ausnehmungen 164 das zweite Anlageelement 158 auf das Kopplungselement 148 aufschieben und parallel zur Längsrichtung des Kopplungselements 148 verschieben zu können.

Bei aufgeschobenem zweiten Anlageelement 158 läßt sich durch Verdrehung, d. h. durch eine Relativbewegung zwischen dem Kopplungselement 148 und dem zweiten Anlageelement 158 quer zur Klemmrichtung, die Haltelasche 162 in Eingriff mit gegenüberliegenden Fixierungsausnehmungen 154 der gegenüberliegenden Eingriffsleisten 150 bringen und somit das zweite Anlageelement 158 an das Kopplungselement 148 koppeln und entsprechend kraftschlüssig fixieren.

Auf diese Weise lassen sich dann zwischen dem ersten Anlageelement 156 und dem zweiten Anlageelement 158 (welches auf die gleiche Weise mit dem Kopplungselement 148 verbunden wird wie das erste Anlageelement) sitzende Knochenplatten fixieren.

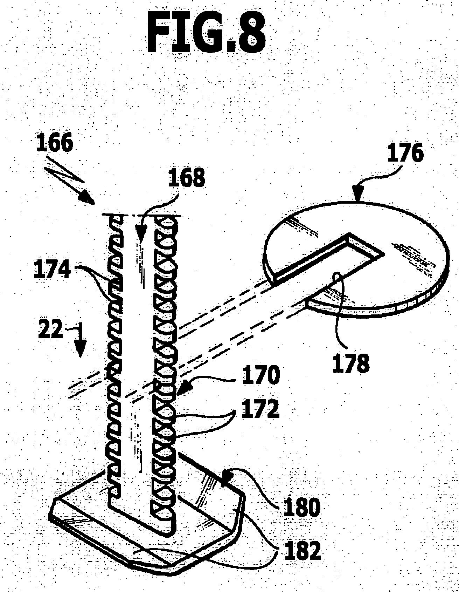

Bei einer achten Ausführungsform 166, welche in Fig. 8 gezeigt ist, weist ein Kopplungselement 168 seitlich gegenüberliegende Eingriffsleisten 170 mit Zähnen 172 und zwischen den Zähnen 172 liegenden Fixierungsausnehmungen 174 auf.

Das zweite Anlageelement 176 ist als Scheibe ausgebildet mit einer rechteckförmigen Ausnehmung 178, die parallel zu einer radialen Richtung orientiert ist. Die Breite der Ausnehmung 178 entspricht dabei im wesentlichen einem Abstand von gegenüberliegenden Fixierungsausnehmungen 174 gegenüberliegender Eingriffsleisten 170. Der Abstand gegenüberliegender Zähne 172 einer Eingriffsleiste 170 entspricht im wesentlichen der Dicke des zweiten Anlageelements 176.

Das zweite Anlageelement 176 läßt sich quer zur Längsrichtung des Kopplungselements 168 auf dieses aufschieben.

Das erste Anlageelement 180 ist bei diesem Ausführungsbeispiel einstückig mit dem Kopplungselement 168 gebildet oder formschlüssig mit diesem verbunden und weist hochgezogene (in Richtung des zweiten Anlageelements 176, wenn dieses an das Kopplungselement 168 gekoppelt ist) Randbereiche 182 auf, welche eine gewisse Elastizität aufweisen. Dadurch lassen sich bei entsprechender Ausübung einer Zugspannung nach Einsatz unter gegenüberliegende Knochenplatten die Randbereiche 182 verformen, dann wird das zweite Anlageelement 176 aufgesetzt und bei Freigabe der Zugspannung bewirkt die von den elastisch verformten Randbereichen 182 auf dazwischenliegende Knochenplatten ausgeübte Kraft eine Erhöhung der Klemmwirkung zwischen dem ersten Anlageelement 180 und dem zweiten Anlageelement 176.

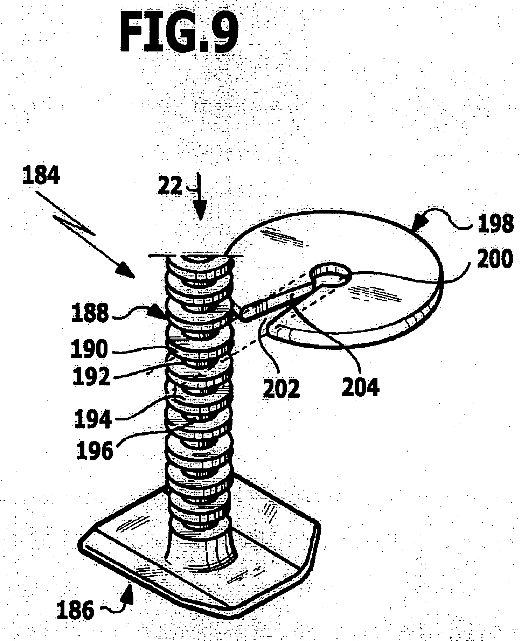

Bei einer neunten Ausführungsform 184, welche in Fig. 9 gezeigt ist, ist das erste Anlageelement 186 wiederum einstückig mit einem Kopplungselement 188 verbunden und im wesentlichen gleich aufgebaut, wie eben für die achte Ausführungsform beschrieben.

Das Kopplungselement 188 selber hat eine zylindrische Gestalt mit zwischen scheibenförmigen Elementen 190 umlaufend gebildeten Fixierungsausnehmungen 192, die dadurch durch gegenüberliegende Ringflächen 194 und eine Zylinderfläche 196, welche an die gegenüberliegenden Ringflächen 194 stößt, begrenzt sind.

Durch die radialsymmetrische Ausbildung des Kopplungselements 188 liegt bezüglich einer Verformung quer zu einer Längsrichtung keine Vorzugsrichtung vor. Dadurch läßt sich das erfindungsgemäße Verbindungselement gemäß der neunten Ausführungsform vorteilhafterweise einsetzen, wenn eine Verformung bewirkt werden soll bzw. erwartet wird.

Das zweite Anlageelement 198 ist als Scheibe ausgebildet mit einer zentralen Öffnung 200. Diese zentrale Öffnung ist an die Zylinderfläche 196 angepaßt, und über sie wird das zweite Anlageelement 198 durch Anlage des Bereiches um die Öffnung an den Ringflächen 194 an dem Kopplungselement 188 gehalten. Von dem Rand des zweiten Anlageelements zur zentralen Öffnung 200 hin weist das zweite Anlageelement 198 eine keilförmige Ausnehmung 202 auf, mittels der das zweite Anlageelement quer zur Längsrichtung des Kopplungselements 188 auf dieses aufschiebbar ist. Dabei laufen Seitenflächen 204 auf die zentrale Öffnung 200 hin aufeinander zu, so daß beim Aufschieben eine Kraft (zur Öffnung dieser Seitenflächen 204) überwunden werden muß, und bei aufgeschobenem zweiten Anlageelement 198 dieses klemmend gehalten wird oder zumindest so, daß der Rückschub behindert ist.

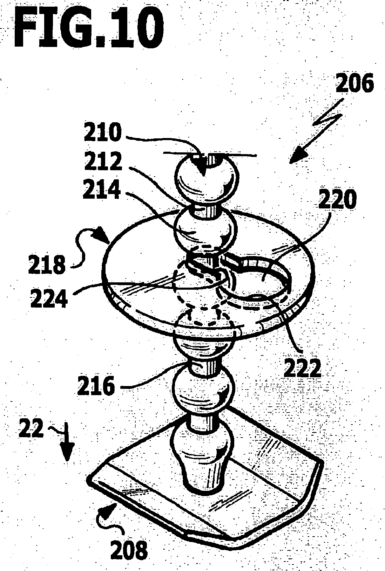

Bei einer zehnten Ausführungsform 206 eines erfindungsgemäßen Verbindungselements, welche in Fig. 10 gezeigt ist, ist ein erstes Anlageelement 208 einstückig mit einem Kopplungselement 210 verbunden. Dieses Kopplungselement weist ein in Längsrichtung verlaufendes zylindrisches Element 212 auf, an welchem kugelförmige Elemente 214 beabstandet angeordnet sind, wobei zwischen benachbarten kugelförmigen Elementen 214 jeweils Fixierungsausnehmungen 216 gebildet sind. Das Kopplungselement 210 selber ist flexibel.

Das zweite Anlageelement 218 weist eine Ausnehmung 220 auf, die eine Durchführungsausnehmung 222 umfaßt, mittels der sich das zweite Anlageelement 218 auf das Kopplungselement 210 aufschieben und relativ zu diesem in dessen Längsrichtung bewegen läßt, und weiter eine verbundene Kopplungsausnehmung 224, durch die sich das zweite Anlageelement 218 auf das Kopplungselement 210 quer zu dessen Längsrichtung aufschieben läßt, um dieses an dem Kopplungselement 210 zu fixieren.

Durch die kugelförmige Ausbildung der Elemente 214 ist eine gute Klemmwirkung auch dann erreichbar, wenn das erste Anlageelement 208 und das zweite Anlageelement 218 nicht parallel ausgerichtet sind, beispielsweise weil die Knochenplatten nicht eben sind, da die Kugelgestalt der Elemente 214 zumindest in gewissem Rahmen auch ein Aufschieben in einer Querrichtung abweichend von einer senkrechten Richtung ermöglicht.

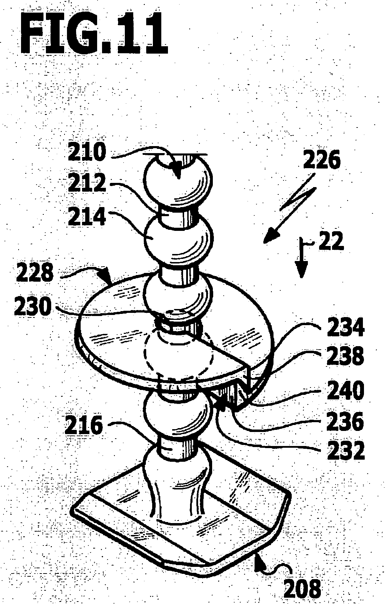

Bei einer elften Ausführungsform 226, welche in Fig. 11 gezeigt ist, ist das Kopplungselement 210 mit dem daran sitzenden ersten Anlageelement 208 gleich ausgebildet wie bei der eben beschriebenen zehnten Ausführungsform.

Ein zweites Anlageelement 228 ist durch einen Ringteller gebildet, welcher eine zentrale Öffnung 230 aufweist und eine Einführungsausnehmung 232. Die Einführungsausnehmung weist gegenüberliegende Seitenflächen 234 und 236 auf, die zueinander höhenversetzt sind. An der Seitenfläche 234 ist ein in Richtung der Seitenfläche 236 weisender Zahn 238 gebildet und an der Seitenfläche 236 ein entsprechender Zahn 240, welcher in Richtung der Seitenfläche 234 weist. Die beiden Zähne 238 und 240 können ineinander verhaken, nachdem das zweite Anlageelement 228 auf das Kopplungselement 210 aufgeschoben ist, um durch diese zusätzliche Verzahnung einen verbesserten Halt zu bewirken.

Insbesondere kann es vorgesehen sein, daß das Überschieben der Zähne 238 und 240 zu deren Verhakung über eine Anlegezange erfolgt.

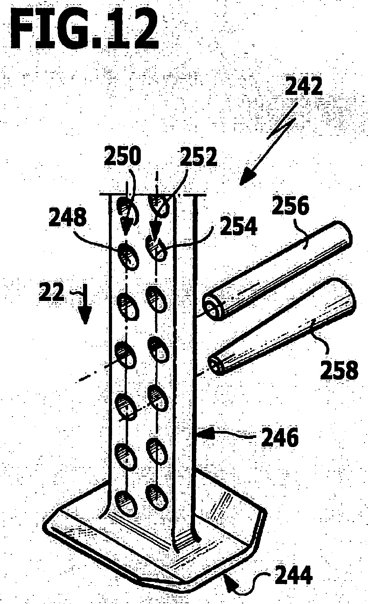

Bei einer zwölften Ausführungsform 242, welche in Fig. 12 gezeigt ist, ist wiederum ein erstes Anlageelement 244 einstückig an einem Kopplungselement 246 gebildet, welches einen rechteckförmigen Querschnitt aufweist. In dem Kopplungselement 246 ist eine erste Reihe von linear ausgerichteten durchgehenden Öffnungen 248 als Eingriffsleiste 250 angeordnet und parallel dazu eine weitere entsprechend ausgebildete Eingriffsleiste 252, wobei die durchgehenden Öffnungen 254 der Eingriffsleiste 252 gegen diejenigen der Eingriffsleiste 250 höhenversetzt sind; dadurch stehen feinere Abstandsstufen zur Ankopplung eines zweiten Anlageelements 256, 258 zur Verfügung.

Eine Öffnung 248 bzw. 254 kann dabei zylindrisch ausgebildet sein, und ein entsprechend dazu angepaßtes zweites Anlageelement 256 ist dann ebenfalls zylindrisch ausgebildet oder weist eine zylindrische Haltelasche auf.

Bei einer Variante dieser Ausführungsform kann es auch vorgesehen sein, daß die Öffnungen 248 bzw. 254 sich in eine Richtung hin verjüngen, beispielsweise kegelstumpfförmig sind. Entsprechend ist dann das zweite Anlageelement 258 auch kegelstumpfförmig ausgebildet. Beim Einschub dieses zweiten Anlageelements 258 in die entsprechenden Öffnungen 248 bzw. 254 läßt sich dann eine Keilwirkung erreichen, welche den Halt des zweiten Anlageelements 258 an dem Kopplungselement 246 verbessert.

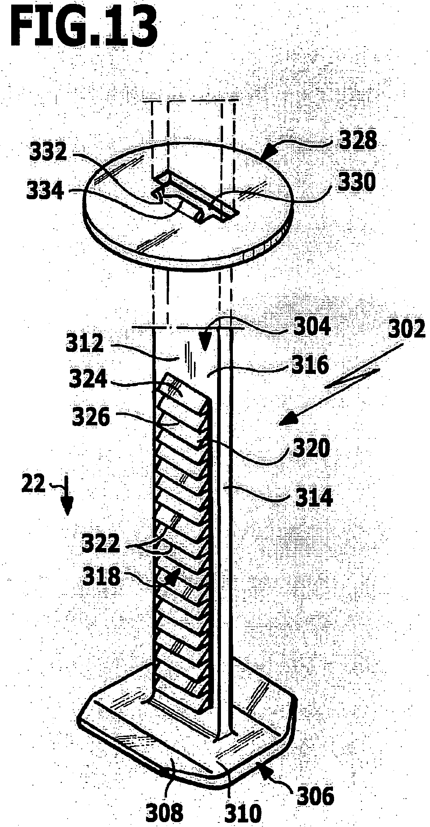

Bei einer 13. Ausführungsform eines erfindungsgemäßen Verbindungselements, welches in Fig. 13 als Ganzes mit 302 bezeichnet ist, ist ein Kopplungselement 304 einstückig mit einem ersten Anlageelement 306 verbunden, welches an einem Ende des Kopplungselements 304 sitzt. Das erste Anlageelement 306 hat die Form einer rechteckigen Platte mit einem hochgezogenen, in Richtung des Kopplungselements 304 weisenden Randbereich 308 auf gegenüberliegenden Seiten des ersten Anlageelements 306. Ein solcher Randbereich 308 weist einen Winkel zu einer Anlagefläche 310 des ersten Anlageelements 306 auf.

Das erste Anlageelement 306 ist aus einem so weit flexiblen Material hergestellt, daß ein Randbereich 308 unter Kraftauswirkung in Richtung dieses Randbereiches verformbar ist, um den Winkel zwischen einem Randbereich 308 und der Anlagefläche 310 zu verringern.

Das Kopplungselement 304 weist einen rechteckigen Querschnitt mit einer längeren Seite 312 und einer schmaleren Seite 314 auf, wobei die längere Seite 312 im wesentlichen parallel zu einem Randbereich 308 ist. Dadurch läßt sich das erfindungsgemäße Verbindungselement 302 über die schmalere Seite 314 in einen Schnittspalt 14 einsetzen, und die gegenüberliegenden Randbereiche 308 des ersten Anlageelements 306 stoßen von unten an zu fixierende Knochenplatten an.

Auf einer Außenfläche 316 des Kopplungselements 304, welche durch die längere Seite 312 in Längsrichtung des Kopplungselements 304 gebildet ist, ist eine Zahnleiste 318 als Eingriffsleiste angeordnet. Die Zahnleiste 318 ist als Rastleiste sägezahnförmig ausgebildet mit im Querschnitt dreieckigen Zähnen 320, die eine Flanke 322 senkrecht zur Außenfläche 316 aufweisen und eine weitere Flanke 324, welche ausgehend von dieser Flanke in Richtung vom ersten Anlageelement 306 weg zur Außenfläche 316 verläuft. Ein solcher Zahn 320 ist dabei im wesentlichen parallel zur Anlagefläche 310 des ersten Anlageelements 306.

Die Zahnleiste 318 umfaßt eine Mehrzahl von hintereinander linear angeordneten Zähnen 320, die insbesondere in einem gleichen Abstand angeordnet sind. Dadurch ist zwischen der Flanke 322 eines bestimmten Zahnes und der Flanke 324 eines benachbarten Zahnes eine Fixierungsausnehmung 326 in der Form einer Rastausnehmung gebildet.

Ein zweites Anlageelement 328 ist scheibenförmig ausgebildet und weist eine Durchgangsöffnung 330 mit rechteckigem Querschnitt auf, welcher an den Querschnitt des Kopplungselements 304 angepaßt ist. Mittels dieser Durchgangsöffnung 330 läßt sich das zweite Anlageelement 328 auf das Kopplungselement 304 aufschieben und sich relativ zu diesem in Längsrichtung des Kopplungselements 304 bewegen. Die Durchgangsöffnung 330 ist mit einer weiteren Ausnehmung 332 verbunden, in der eine Haltelasche 334 zum Eingriff in eine Fixierungsausnehmung 326 sitzt. Die Haltelasche 324 weist zur Durchgangsöffnung 330 hin und ist insbesondere derart flexibel, daß sie über eine Flanke 324 eines Zahns 320 schiebbar ist und dann in eine Fixierungsausnehmung 326 nach Überschub über die Flanke einrasten kann. Dies bedeutet, daß zum Einrasten die Haltelasche 334 eine Bewegung quer zur Längsrichtung des Kopplungselements 304 ausführt, wenn die Haltelasche von einer verformten Stellung durch Andruck an der schrägen Flanke 324 in ihre unverformte Stellung in der Fixierungsausnehmung 326 zurückkehrt. Die Relativbewegung zwischen Haltelasche 334 und Kopplungselement 304 erfolgt damit aus einer nicht verriegelten Stellung in eine verriegelte Stellung.

Die Haltelasche 334 ist bevorzugterweise in einer Ebene mit einer oberen und unteren Fläche des zweiten Anlageelements 328 angeordnet.

Zur Fixierung von zwischen dem ersten Anlageelement 306 und dem zweiten Anlageelement 328 benachbart liegenden Knochenplatten, wobei das Kopplungselement 304 in einem Schnittspalt 14 liegt, wird das zweite Anlageelement 328 auf das Kopplungselement 304 aufgeschoben und die Haltelasche 334 über die Zahnleiste 380 geschoben. Durch eine in einer Fixierungsausnehmung 326 eintauchende Haltelasche 334 ist das zweite Anlageelement 328 kraftschlüssig an dem Kopplungselement 304 fixiert. Dadurch läßt sich zwischen dem ersten Anlageelement 306 und dem zweiten Anlageelement 328 eine Klemmkraft auf die Knochenplatten ausüben, die dadurch fixiert werden. Durch die hochgezogenen Randbereiche 308, die sich unter Zugspannung bei der Anlage an die Knochenplatten elastisch verformen und dementsprechend unter Vorspannung stehen, wird diese Klemmkraft verstärkt und damit die Fixierung verbessert.

Es kann vorgesehen sein, daß das Kopplungselement eine Sollbruchstelle aufweist, wie sie bereits im Zusammenhang mit der sechsten Ausführungsform beschrieben wurde.

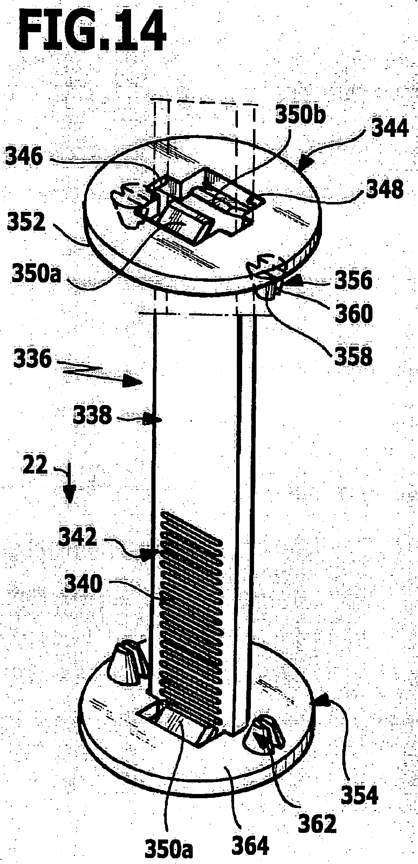

Bei einer 14. Ausführungsform 336, welche in Fig. 14 gezeigt ist, umfaßt ein Kopplungselement 338 durchgehende Ausnehmungen 340, welche einen rechteckförmigen Querschnitt haben und in der Form einer Eingriffsleiste 342 parallel zueinander beabstandet angeordnet sind.

Ein zweites Anlageelement 344 ist scheibenförmig ausgebildet mit einer rechteckförmigen Durchgangsöffnung 346, mittels welcher das zweite Anlageelement 344 auf das Kopplungselement 338 aufschiebbar ist. Weiterhin ist quer zur Durchgangsöffnung eine Ausnehmung 348 in dem zweiten Anlageelement 344 gebildet, an der gegenüberliegend Haltelaschen 350a, 350b sitzen, die zum Eingriff in die Ausnehmungen 340, welche Fixierungsausnehmungen sind, dienen. Eine solche Haltelasche 350a, 350b hat die Form einer Federzunge, welche in einem Winkel zu einer Anlagefläche 352 des zweiten Anlageelements 344 abgewandt von dieser steht.

Auf der Anlagefläche 352 weist das zweite Anlageelement zwei Haltekörper 356 auf, so daß diese einem ersten Anlageelement 354 zugewandt in einem Schnittspalt 14 zwischen benachbarten Knochenplatten positionierbar sind. Die zwei Haltekörper 356 liegen dabei auf einer Verbindungslinie, welche auf einem Durchmesser des zweiten Anlageelements 344 liegt. Ein Haltekörper 356 selber umfaßt zwei gegenüberliegend angeordnete Halteelemente 358, welche kegelstumpfförmig ausgebildet sind. Zwischen diesen beiden Halteelementen 358 ist ein Zwischenraum 360. Dadurch lassen sich die Halteelemente 358 aufeinander zu bewegen, bis der Zwischenraum 360 überbrückt ist und die beiden Halteelemente 358 sich berühren.

Solch ausgebildete Haltekörper 356 sorgen für eine zusätzliche Verklemmung, und die Fixierung benachbarter Knochenplatten wird verbessert. Durch die zwei Halteelemente 358 eines Haltekörpers und den Zwischenraum 360 lassen sich dabei die Knochenplatten noch in gewissem Rahmen aufeinander zu bewegen, so daß Korrekturen bezüglich der Ausrichtung durchführbar sind.

Ähnlich wie bereits für die 13. Ausführungsform beschrieben, wird das zweite Anlageelement 344 auf das Kopplungselement 338 aufgeschoben und die beiden Haltelaschen 350a und 350b greifen gleichzeitig in eine entsprechende Ausnehmung 340 ein, um das zweite Anlageelement 344 an dem Kopplungselement 338 zu fixieren.

Das erste Anlageelement 354 ist grundsätzlich gleich aufgebaut wie das zweite Anlageelement 344, jedoch sind zugehörige Haltekörper 362 an einer Anlagefläche 364 angeordnet, welche die gegenüberliegende Fläche zu der Anlagefläche 352 des zweiten Anlageelements 344 ist; damit sind das erste Anlageelement 354 und das zweite Anlageelement 344 spiegelsymmetrisch zueinander.

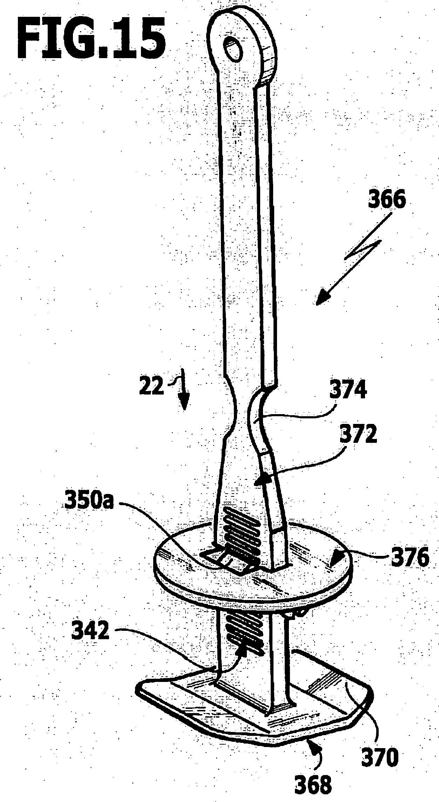

Bei einer 15. Ausführungsform 366, welche in Fig. 15 gezeigt ist, ist ein erstes Anlageelement 368 mit hochgezogenen Randbereichen 370 einstückig mit einem Kopplungselement 372 verbunden. Dieses Kopplungselement 372 weist eine wie oben beschriebene Sollbruchstelle 374 auf. Ein zweites Anlageelement 376 ist wie das zweite Anlageelement 344 der 14. Ausführungsform 336 aufgebaut.

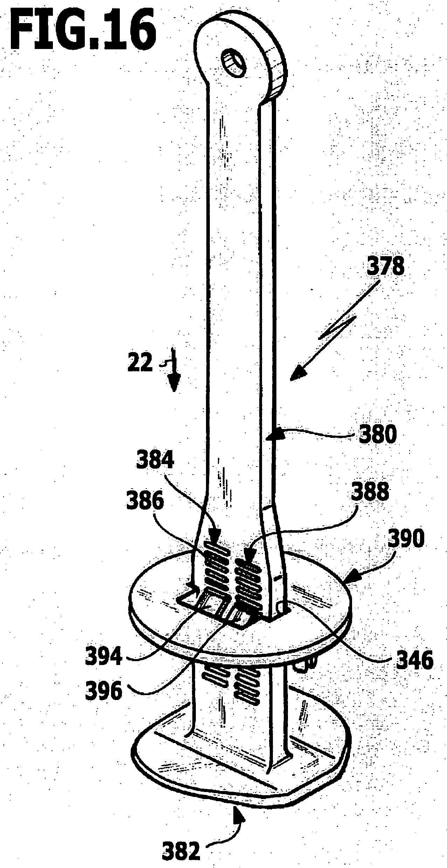

Bei einer Variante der 15. Ausführungsform, welche in Fig. 16 als Ganzes mit 378 bezeichnet ist (16. Ausführungsform), weist ein Kopplungselement 380, an welchem einstückig ein erstes Anlageelement 382 sitzt, eine erste Eingriffsleiste 384 mit Durchgangsausnehmungen 386 und eine parallel dazu angeordnete zweite Eingriffsleiste 388 auf, welche jedoch in Längsrichtung des Kopplungselements 380 so versetzt angeordnet ist, daß zwischen benachbarten Durchgangsöffnungen der ersten Eingriffsleiste, bezogen auf eine Höhe in Längsrichtung des ersten Kopplungselements 380, eine Durchgangsausnehmung der zweiten Eingriffsleiste 388 sitzt. Dadurch läßt sich eine feinere Höhenverstellung eines zweiten Anlageelements 390 gegenüber dem ersten Anlageelement 382 erreichen.

Das zweite Anlageelement wiederum weist, wie bereits oben für die 14. Ausführungsform beschrieben, eine Durchgangsöffnung 346 auf. In einer Ausnehmung 392 quer dazu sitzen jedoch auf gegenüberliegenden Seiten jeweils zwei Haltelaschen 394 und 396, wobei die Haltelasche 394 zum Eingriff in die erste Eingriffsleiste 384 dient und entsprechend die zweite Haltelasche 396 zum Eingriff in die zweite Eingriffsleiste 388. Das zweite Anlageelement 390 weist damit insgesamt vier als Federzungen ausgebildete Haltelaschen auf, die überkreuz auf beiden Seiten der Eingriffsleisten 384 und 388 eingreifen können.

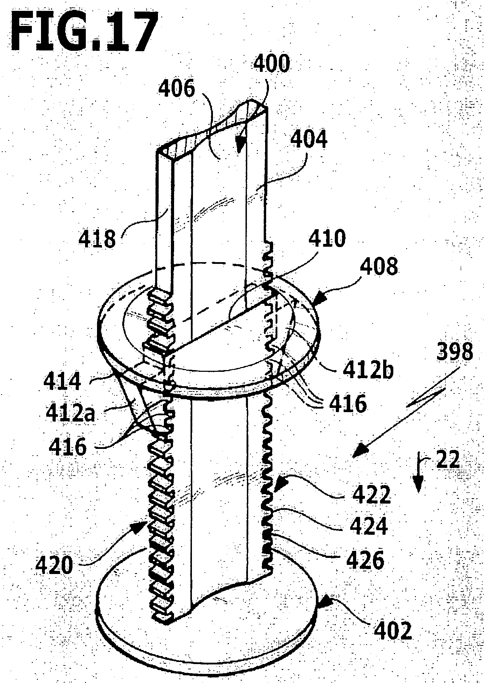

Bei einer 17. Ausführungsform 398, welche in Fig. 17 gezeigt ist und nicht unter die Ansprüche fällt, sitzt an einem Kopplungselement 400 beispielsweise einstückig oder formschlüssig ein erstes scheibenförmiges Anlageelement 402 und weist einen im wesentlichen rechteckigen Querschnitt auf, wobei in den gegenüberliegenden längeren Seiten 404 mittig eine Vertiefung 406 in der Form einer Eindellung gebildet ist.

Ein zweites Anlageelement 408 ist ebenfalls scheibenförmig ausgebildet mit einer Durchgangsausnehmung 410, welche einen rechteckförmigen Querschnitt hat, so daß das zweite Anlageelement 408 auf das Kopplungselement 400 aufschiebbar ist. An dem zweiten Anlageelement 408 sitzen auf einem Durchmesser gegenüberliegend Haltelaschen 412a und 412b, welche über eine Anlagefläche 414 des zweiten Anlageelements 408 hinausstehen. Eine solche Haltelasche 412a bzw. 412b ist der anderen Haltelasche zugewandt mit einer Mehrzahl von Zähnen 416 versehen, beispielsweise mit drei Zähnen, welche beabstandet angeordnet sind.

Das Kopplungselement 400 weist gegenüberliegend auf gegenüberliegenden Seitenflächen 418 eine erste Eingriffsleiste 420 und eine zweite Eingriffsleiste 422 auf, die jeweils mit Zähnen 424 versehen sind, wobei zwischen benachbarten Zähnen einer Eingriffsleiste 420 bzw. 422 eine Fixierungsausnehmung 426 zum Eingriff jeweils eines Zahns 416 des zweiten Anlageelements 408 gebildet ist. Dadurch sind die Eingriffsleisten 420 und 422 im Schnittspalt 14 positionierbar, so daß sie nicht an benachbarten Knochenplatten anliegen. Es kann vorgesehen sein, daß die beiden Eingriffsleisten 420 und 422 so höhenversetzt zueinander angeordnet sind, daß die Zähne 424 der beiden Eingriffsleisten relativ zueinander höhenversetzt sind, um eine feinere Höhenverstellbarkeit zu erreichen.

Durch die Mehrzahl von Zähnen einer Haltelasche 412a bzw. 412b greifen gleichzeitig auch eine Mehrzahl von Zähnen in die erste Eingriffsleiste 420 und die zweite Eingriffsleiste 422 ein, um so eine sichere Fixierung zu erhalten.

Um das zweite Anlageelement 408 auf dem Kopplungselement 400 zu verschieben bzw. die Fixierung zu lösen, sind die Haltelaschen 412a bzw. 412b derart flexibel ausgebildet und an dem zweiten Anlageelement 408 angeordnet, daß sie zur Aufhebung der Kopplung von den Eingriffsleisten 420 bzw. 422 weg beweglich sind. Eine dabei stattfindende Verformung des zweiten Anlageelements 408, die zu einer - unerwünschten - Verklemmung an dem Kopplungselement 400 führen könnte, ist dabei durch die Vertiefungen 406 aufnehmbar, so daß die Verschieblichkeit des zweiten Anlageelements 408 längs des Kopplungselements 400 nicht beeinträchtigt wird.

## Patentansprüche

1. Chirurgisches Verbindungselement zur Fixierung benachbart angeordneter Knochenplatten, umfassend ein erstes Anlageelement (306; 368; 382), ein zweites Anlageelement (328; 376; 390) und ein Kopplungselement (372; 380), mittels welchem erstes und zweites Anlageelement derart miteinander koppelbar sind, daß zwischen dem ersten und zweiten Anlageelement liegende Knochenplatten (10, 12) fixierbar sind, wobei das Kopplungselement (304; 372; 380) mindestens eine Fixierungsausnehmung (326; 386) aufweist, in die ein entsprechendes Halteelement (334; 350a; 394; 396) des zweiten Anlageelements (328; 376; 390) zur Kopplung dieses zweiten Anlageelements (328; 376; 390) an das Kopplungselement (304; 372; 380) eingreifbar ist, **dadurch gekennzeichnet** , daßeinHalteelement(334; 350a; 394; 396) eine Haltelasche ist und daß das erste Anlageelement (306; 368; 382) und/oder zweite Anlageelement (328; 376; 390) einen hochgezogenen Randbereich (308; 370) zur Erzeugung einer Zugspannung in einer Klemmrichtung (22) aufweist, wobei der hochgezogene Randbereich (308; 370) einen Winkel zu einer Anlagefläche (310) des entsprechenden Anlageelements (306; 368; 382) aufweist, elastisch verformbar ist, um den Winkel zwischen dem Randbereich (308; 370) und der Anlagefläche (310) zu verringern, und dadurch in Klemmstellung eine Kraft ausübt, welche die Klemmung verbessert.

2. Chirurgisches Verbindungselement nach Anspruch 1, **dadurch gekennzeichnet, daß** der Eingriff im wesentlichen quer zu der Klemmrichtung (22) erfolgt.

3. Chirurgisches Verbindungselement nach Anspruch 1 oder 2, **dadurch gekennzeichnet, daß** das zweite Anlageelement (376; 390) kraftschlüssig an dem Kopplungselement fixierbar ist.

4. Chirurgisches Verbindungselement nach einem der vorangehenden Ansprüche, **dadurch gekennzeichnet, daß** das zweite Anlageelement (376; 390) und das Kopplungselement (372; 380) so ausgebildet und aufeinander abgestimmt sind, daß durch Relativbewegung zwischen dem Kopplungselement (372; 380) und dem zweiten Anlageelement (376; 390) quer zur Klemmrichtung (22) die Knochenplatten (10, 12) zwischen dem ersten Anlageelement (368; 382) und dem zweiten Anlageelement (376; 390) fixierbar sind.

5. Chirurgisches Verbindungselement nach einem der vorangehenden Ansprüche, **dadurch gekennzeichnet, daß** eine Fixierungsstellung des ersten Anlageelements (368; 382) und des zweiten Anlageelements (376; 390) durch Relativbewegung zwischen dem Kopplungselement (372; 380) und dem zweiten Anlageelement (376; 390) quer zur Klemmrichtung (22) verriegelbar ist.

6. Chirurgisches Verbindungselement nach einem der vorangehenden Ansprüche, **dadurch gekennzeichnet, daß** eine Fixierungsstellung des ersten Anlageelements (368; 382) und des zweiten Anlageelements (376; 390) durch Relativbewegung zwischen dem Kopplungselement (372; 380) und dem zweiten Anlageelement (376; 380) quer zur Klemmrichtung (22) entriegelbar ist.

7. Chirurgisches Verbindungselement nach einem der vorangehenden Ansprüche, **dadurch gekennzeichnet, daß** das erste Anlageelement (368; 382) und/oder zweite Anlageelement (376; 390) einen Haltekörper (356) aufweisen, welcher in einen Zwischenraum (14) zwischen benachbart angeordneten Knochenplatten (10, 12) einsetzbar ist.

8. Chirurgisches Verbindungselement nach Anspruch 7, **dadurch gekennzeichnet, daß** ein Haltekörper (356) in Querrichtung zur Klemmrichtung (22) elastisch ausgebildet ist.

9. Chirurgisches Verbindungselement nach einem der vorangehenden Ansprüche, **dadurch gekennzeichnet, daß** das Verbindungselement aus einem körperverträglichen Material hergestellt ist.

10. Chirurgisches Verbindungselement nach einem der vorangehenden Ansprüche, **dadurch gekennzeichnet, daß** das Verbindungselement aus einem resorbierbaren Kunststoffmaterial gefertigt ist.

11. Chirurgisches Verbindungselement nach einem der vorangehenden Ansprüche, **dadurch gekennzeichnet, daß** das Kopplungselement (372) mit einer Sollbruchstelle (374) versehen ist.

12. Chirurgisches Verbindungselement nach einem der vorangehenden Ansprüche, **dadurch gekennzeichnet, daß** das erste Anlageelement (368; 390) einstückig oder formschlüssig mit dem Kopplungselement (372; 380) verbunden ist.

13. Chirurgisches Verbindungselement nach einem der vorangehenden Ansprüche, **dadurch gekennzeichnet, daß** das zweite Anlageelement (376; 390) mindestens eine Durchgangsausnehmung (346) für das Kopplungselement (372; 380) aufweist, so daß das zweite Anlageelement (376; 390) und das Kopplungselement (372; 380) relativ zueinander im wesentlichen parallel zur Klemmrichtung (22) beweglich sind.

14. Chirurgisches Verbindungselement nach einem der vorangehenden Ansprüche, **dadurch gekennzeichnet, daß** das zweite Anlageelement (376; 390) mit dem Kopplungselement (372; 380) durch eine Relativbewegung eines am zweiten Anlageelement (376; 390) sitzenden Halteelements (350a; 394; 396) zum Kopplungselement (372; 380) mit diesem verbindbar ist.

15. Chirurgisches Verbindungselement nach einem der vorangehenden Ansprüche, **dadurch gekennzeichnet, daß** das Kopplungselement (372; 380) ein starrer Körper ist.

16. Chirurgisches Verbindungselement nach einem der vorangehenden Ansprüche, **dadurch gekennzeichnet, daß** das Kopplungselement (372; 380) so dimensioniert ist, daß es in einer Querrichtung zu seiner Längsrichtung eine größere Breite aufweist als in der Querrichtung senkrecht dazu.

17. Chirurgisches Verbindungselement nach einem der vorangehenden Ansprüche, **dadurch gekennzeichnet, daß** das Kopplungselement (372; 380) zumindest in dem Bereich, welcher zwischen benachbarten Knochenplatten positionierbar ist, einen im wesentlichen rechteckigen Querschnitt aufweist.

18. Chirurgisches Verbindungselement nach einem der vorangehenden Ansprüche, **dadurch gekennzeichnet, daß** das Halteelement (350a; 394; 396) des zweiten Anlageelements (376; 390) quer zur Klemmrichtung (22) in eine Fixierungsausnehmung (386) eingreift.

19. Chirurgisches Verbindungselement nach einem der vorangehenden Ansprüche, **dadurch gekennzeichnet, daß** das Kopplungselement (372; 380) eine Mehrzahl von Fixierungsausnehmungen (386) umfaßt, welche in Längsrichtung des Kopplungselements (372; 380) angeordnet sind.

20. Chirurgisches Verbindungselement nach einem der vorangehenden Ansprüche, **dadurch gekennzeichnet, daß** benachbarte Fixierungsausnehmungen (386) in gleichem Abstand zueinander angeordnet sind.

21. Chirurgisches Verbindungselement nach einem der vorangehenden Ansprüche, **dadurch gekennzeichnet, daß** das Kopplungselement (372; 380) mit einer aus Fixierungsausnehmungen gebildeten Eingriffsleiste (384; 388) versehen ist.

22. Chirurgisches Verbindungselement nach Anspruch 21, **dadurch gekennzeichnet, daß** die Eingriffsleiste (384; 388) als Rastleiste ausgebildet ist.

23. Chirurgisches Verbindungselement nach Anspruch 21 oder 22, **dadurch gekennzeichnet, daß** das Kopplungselement (380) mindestens zwei Eingriffsleisten (384, 388) aufweist.

24. Chirurgisches Verbindungselement nach Anspruch 23, **dadurch gekennzeichnet, daß** mindestens zwei Eingriffsleisten (384, 388) bezüglich des Abstands von Fixierungsausnehmungen (386) relativ zum ersten Anlageelement (382) versetzt zueinander angeordnet sind.

25. Chirurgisches Verbindungselement nach einem der vorangehenden Ansprüche, **dadurch gekennzeichnet, daß** eine Seitenfläche des Kopplungselements mit einer Eingriffsleiste versehen ist.

26. Chirurgisches Verbindungselement nach einem der vorangehenden Ansprüche, **dadurch gekennzeichnet, daß** eine Eingriffsleiste (384; 388) zwischen Seitenflächen des Kopplungselements (372; 380) am Kopplungselement (372; 380) angeordnet ist.

27. Chirurgisches Verbindungselement nach einem der Ansprüche 22 bis 26, **dadurch gekennzeichnet, daß** eine Eingriffsleiste stufenförmig ausgebildet ist.

28. Chirurgisches Verbindungselement nach einem der Ansprüche 22 bis 27, **dadurch gekennzeichnet, daß** eine Eingriffsleiste sägezahnförmig ausgebildet ist.

29. Chirurgisches Verbindungselement nach einem der vorangehenden Ansprüche, **dadurch gekennzeichnet, daß** eine Eingriffsleiste (384; 388) rechteckförmige Ausnehmungen (386) als Fixierungsausnehmungen umfaßt.

30. Chirurgisches Verbindungselement nach einem der vorangehenden Ansprüche, **dadurch gekennzeichnet, daß** eine Fixierungsausnehmung einen sich verjüngenden Querschnitt aufweist.

31. Chirurgisches Verbindungselement nach einem der vorangehenden Ansprüche, **dadurch gekennzeichnet, daß** das zweite Anlageelement (376; 390) eine Haltelasche (394; 396) aufweist, welche in eine Fixierungsausnehmung (386) eintauchbar ist und mittels der sich das zweite Anlageelement (376; 390) mit dem Kopplungselement (372; 380) verbinden läßt.

32. Chirurgisches Verbindungselement nach Anspruch 31, **dadurch gekennzeichnet, daß** eine Haltelasche eine Mehrzahl von Haltezähnen zum gleichzeitigen Eingriff in eine Mehrzahl von Fixierungsausnehmungen umfaßt.

33. Chirurgisches Verbindungselement nach Anspruch 31 oder 32, **dadurch gekennzeichnet, daß** eine Haltelasche (350a; 350b) in einem Winkel zu einer Anlagefläche (352) des zweiten Anlageelements (344) angeordnet ist.

34. Chirurgisches Verbindungselement nach einem der vorangehenden Ansprüche, **dadurch gekennzeichnet, daß** die Relativbewegung zwischen dem zweiten Anlageelement (376; 390) und dem Kopplungselement (372; 380) zur Fixierung des zweiten Anlageelements (376; 390) mit dem Kopplungselement (372; 380) ausgehend von einer nicht fixierten Stellung in eine Fixierstellung erfolgt.

35. Chirurgisches Verbindungselement nach einem der vorangehenden Ansprüche, **dadurch gekennzeichnet, daß** benachbarte Fixierungsausnehmungen (386) getrennt voneinander sind.

36. Chirurgisches Verbindungselement nach einem der vorangehenden Ansprüche, **dadurch gekennzeichnet, daß** benachbarte Fixierungsausnehmungen (386) nicht miteinander verbunden sind.

## Claims

1. Surgical connecting element for fixing adjacently arranged bone plates comprising a first bearing element (306; 368; 382), a second bearing element (328; 376; 390) and a coupling element (372; 380) by means of which the first and second bearing element are couplable to one another in such a manner that bone plates (10, 12) located between the first and second bearing element are fixable, wherein the coupling element (304; 372; 380) comprises at least one fixing recess (326; 386) in which a corresponding holding element (334; 350a; 394; 396) of the second bearing element (328; 376; 390) is engageable for the purposes of coupling this second bearing element (328; 376; 390) to the coupling element (304; 372; 380),
**characterized in that** a holding element (334; 350a; 394; 396) is a holding lug and **in that** the first bearing element (306; 368; 382) and/or second bearing element (328; 376; 390) comprises a raised edge region (308; 370) for producing a tensile stress in a clamping direction (22), wherein the raised edge region (308; 370) is at an angle to a bearing surface (310) of the corresponding bearing element (306; 368; 382) and is flexibly deformable in order to reduce the angle between the edge region (308; 370) and the bearing surface (310) and thereby exert a force in a clamping position which improves the clamping process.

2. Surgical connecting element in accordance with Claim 1, **characterized in that** the engagement process is effected substantially transversely to the clamping direction (22).

3. Surgical connecting element in accordance with Claim 1 or 2, **characterized in that** the second bearing element (376; 390) is fixable to the coupling element in force-locking manner.

4. Surgical connecting element in accordance with any of the preceding Claims, **characterized in that** the second bearing element (376; 390) and the coupling element (372; 380) are formed and matched to one another in such a way that the bone plates (10, 12) are fixable between the first bearing element (368; 382) and the second bearing element (376; 390) by relative movement between the coupling element (372; 380) and the second bearing element (376; 390) transversely to the clamping direction (22).

5. Surgical connecting element in accordance with any of the preceding Claims, **characterized in that** a fixing position of the first bearing element (368; 382) and the second bearing element (376; 390) is lockable by relative movement between the coupling element (372; 380) and the second bearing element (376; 390) transversely to the clamping direction (22).

6. Surgical connecting element in accordance with any of the preceding Claims, **characterized in that** a fixing position of the first bearing element (368; 382) and the second bearing element (376; 390) is unlockable by relative movement between the coupling element (372; 380) and the second bearing element (376; 380) transversely to the clamping direction (22).

7. Surgical connecting element in accordance with any of the preceding Claims, **characterized in that** the first bearing element (368; 382) and/or second bearing element (376; 390) comprise a holding body (356) which is insertible into an intermediate space (14) between adjacently arranged bone plates (10, 12).

8. Surgical connecting element in accordance with Claim 7, **characterized in that** a holding body (356) is resilient in a direction transverse to the clamping direction (22).

9. Surgical connecting element in accordance with any of the preceding Claims, **characterized in that** the connecting element is made of a bio-compatible material.

10. Surgical connecting element in accordance with any of the preceding Claims, **characterized in that** the connecting element is fabricated from a resorbable synthetic material.

11. Surgical connecting element in accordance with any of the preceding Claims, **characterized in that** the coupling element (372) is provided with a predetermined breaking point (374).

12. Surgical connecting element in accordance with any of the preceding Claims, **characterized in that** the first bearing element (368; 390) is connected to the coupling element (372; 380) in one-piece or positive-locking manner.

13. Surgical connecting element in accordance with any of the preceding Claims, **characterized in that** the second bearing element (376; 390) comprises at least one passage recess (346) for the coupling element (372; 380) so that the second bearing element (376; 390) and the coupling element (372; 380) are moveable relative to each other substantially parallel to the clamping direction (22).

14. Surgical connecting element in accordance with any of the preceding Claims, **characterized in that** the second bearing element (376; 390) is connectible to the coupling element (372; 380), by means of a relative movement of a holding element (350a; 394; 396) seated on the second bearing element (376; 390) with respect to the coupling element (372; 380).

15. Surgical connecting element in accordance with any of the preceding Claims, **characterized in that** the coupling element (372; 380) is a rigid body.

16. Surgical connecting element in accordance with any of the preceding Claims, **characterized in that** the coupling element (372; 380) is dimensioned such that it is of greater width in one transverse direction to its longitudinal direction than in the transverse direction perpendicular thereto.

17. Surgical connecting element in accordance with any of the preceding Claims, **characterized in that** the coupling element (372; 380) has a substantially rectangular cross section at least in the region which is positionable between adjacent bone plates.

18. Surgical connecting element in accordance with any of the preceding Claims, **characterized in that** the holding element (350a; 394; 396) of the second bearing element (376; 390) engages in a fixing recess (386) transversely to the clamping direction (22).

19. Surgical connecting element in accordance with any of the preceding Claims, **characterized in that** the coupling element (372; 380) comprises a plurality of fixing recesses (386) which are arranged in the longitudinal direction of the coupling element (372; 380).

20. Surgical connecting element in accordance with any of the preceding Claims, **characterized in that** adjacent fixing recesses (386) are arranged at the same spacing from one another.

21. Surgical connecting element in accordance with any of the preceding Claims, **characterized in that** the coupling element (372; 380) is provided with an engagement strip (384; 388) formed of fixing recesses.

22. Surgical connecting element in accordance with Claim 21, **characterized in that** the engagement strip (384; 388) is in the form of a latching strip.

23. Surgical connecting element in accordance with Claim 21 or 22, **characterized in that** the coupling element (380) comprises at least two engagement strips (384, 388).

24. Surgical connecting element in accordance with Claim 23, **characterized in that** at least two engagement strips (384, 388) are off-set from one another with respect to the spacing of the fixing recesses (386) relative to the first bearing element (382).

25. Surgical connecting element in accordance with any of the preceding Claims, **characterized in that** a side surface of the coupling element is provided with an engagement strip.

26. Surgical connecting element in accordance with any of the preceding Claims, **characterized in that** an engagement strip (384; 388) is arranged on the coupling element (372; 380) between side surfaces of the coupling element (372; 380).

27. Surgical connecting element in accordance with any of the Claims 22 to 26, **characterized in that** an engagement strip is in the form of steps.

28. Surgical connecting element in accordance with any of the Claims 22 to 27, **characterized in that** an engagement strip is in the form of saw teeth.

29. Surgical connecting element in accordance with any of the preceding Claims, **characterized in that** an engagement strip (384; 388) comprises fixing recesses in the form of rectangular recesses (386).

30. Surgical connecting element in accordance with any of the preceding Claims, **characterized in that** a fixing recess has a tapering cross section.

31. Surgical connecting element in accordance with any of the preceding Claims, **characterized in that** the second bearing element (376; 390) comprises a holding lug (394; 396) which is insertible into a fixing recess (386) and by means of which the second bearing element (376; 390) can be connected to the coupling element (372; 380).

32. Surgical connecting element in accordance with Claim 31, **characterized in that** a holding lug comprises a plurality of holding teeth for simultaneous engagement in a plurality of fixing recesses.

33. Surgical connecting element in accordance with Claim 31 or 32, **characterized in that** a holding lug (350a; 350b) is arranged at an angle to a bearing surface (352) of the second bearing element (344).

34. Surgical connecting element in accordance with any of the preceding Claims, **characterized in that** the relative movement between the second bearing element (376; 390) and the coupling element (372; 380) for fixing the second bearing element (376; 390) to the coupling element (372; 380) is effected by movement from a non-fixed position into a fixing position.

35. Surgical connecting element in accordance with any of the preceding Claims, **characterized in that** adjacent fixing recesses (386) are separated from one another.

36. Surgical connecting element in accordance with any of the preceding Claims, **characterized in that** adjacent fixing recesses (386) are not connected to one another.

## Revendications

1. Elément de liaison chirurgical destiné à fixer des plaques osseuses adjacentes, comprenant un premier élément d'appui (306 ; 368 ; 382), un deuxième élément d'appui (328 ; 376 ; 390) et un élément de couplage (372 ; 380), au moyen duquel le premier et le deuxième élément d'appui peuvent être couplés l'un à l'autre de manière à pouvoir fixer les plaques osseuses (10, 12) se trouvant entre le premier et le deuxième élément d'appui, l'élément de couplage (304 ; 372 ; 380) comprenant au moins un évidement de fixation (326 ; 386), dans lequel un élément de retenue (334 ; 350a ; 394 ; 396) correspondant du deuxième élément d'appui (328 ; 376 ; 390) peut être inséré pour coupler ce deuxième élément d'appui (328 ; 376 ; 390) à l'élément de couplage (304 ; 372 ; 380), **caractérisé en ce qu'**un élément de retenue (334 ; 350a ; 394 ; 396) est une languette de retenue et **en ce que** le premier élément d'appui (306 ; 368 ; 382) et/ou le deuxième élément d'appui (328 ; 376 ; 390) présentent une zone marginale surélevée (308 ; 370) destinée à produire une contrainte de traction dans une direction de serrage (22), la zone marginale surélevée (308 ; 370) formant un angle avec une surface d'appui (310) de l'élément d'appui (306 ; 368 ; 382) correspondant, étant déformable élastiquement, afin de réduire l'angle entre la zone marginale (308 ; 370) et la surface d'appui (310), et exerçant ainsi dans la position de serrage une force qui améliore le serrage.

2. Elément de liaison chirurgical selon la revendication 1, **caractérisé en ce que** l'insertion est réalisée sensiblement transversalement à la direction de serrage (22).

3. Elément de liaison chirurgical selon la revendication 1 ou 2, **caractérisé en ce que** le deuxième élément d'appui (376 ; 390) peut être fixé à force sur l'élément de couplage.

4. Elément de liaison chirurgical selon l'une quelconque des revendications précédentes, **caractérisé en ce que** le deuxième élément d'appui (376 ; 390) et l'élément de couplage (372 ; 380) sont conçus et adaptés l'un à l'autre de telle sorte que, sous l'effet d'un mouvement relatif entre l'élément de couplage (372 ; 380) et le deuxième élément d'appui (376 ; 390) transversalement à la direction de serrage (22), les plaques osseuses (10, 12) peuvent être fixées entre le premier élément d'appui (368 ; 382) et le deuxième élément d'appui (376 ; 390).

5. Elément de liaison chirurgical selon l'une quelconque des revendications précédentes, **caractérisé en ce qu'**une position de fixation du premier élément d'appui (368 ; 382) et du deuxième élément d'appui (376 ; 390) peut être verrouillée par un mouvement relatif entre l'élément de couplage (372 ; 380) et le deuxième élément d'appui (376 ; 390) transversalement à la direction de serrage (22).

6. Elément de liaison chirurgical selon l'une quelconque des revendications précédentes, **caractérisé en ce qu'**une position de fixation du premier élément d'appui (368 ; 382) et du deuxième élément d'appui (376 ; 390) peut être déverrouillée par un mouvement relatif entre l'élément de couplage (372 ; 380) et le deuxième élément d'appui (376 ; 390) transversalement à la direction de serrage (22).

7. Elément de liaison chirurgical selon l'une quelconque des revendications précédentes, **caractérisé en ce que** le premier élément d'appui (368 ; 382) et/ou le deuxième élément d'appui (376 ; 390) présentent un corps de retenue (356), lequel peut être inséré dans un intervalle (14) entre les plaques osseuses (10, 12) adjacentes.

8. Elément de liaison chirurgical selon la revendication 7, **caractérisé en ce qu'**un corps de retenue (356) est conçu de manière à être élastique dans la direction transversale à la direction de serrage (22).

9. Elément de liaison chirurgical selon l'une quelconque des revendications précédentes, **caractérisé en ce que** l'élément de liaison est fabriqué en un matériau biocompatible.

10. Elément de liaison chirurgical selon l'une quelconque des revendications précédentes, **caractérisé en ce que** l'élément de liaison est réalisé en une matière plastique résorbable.

11. Elément de liaison chirurgical selon l'une quelconque des revendications précédentes, **caractérisé en ce que** l'élément de couplage (372) est pourvu d'un point de rupture (374).

12. Elément de liaison chirurgical selon l'une quelconque des revendications précédentes, **caractérisé en ce que** le premier élément d'appui (368 ; 382) est relié d'une seule pièce ou par coopération de formes à l'élément de couplage (372 ; 380).

13. Elément de liaison chirurgical selon l'une quelconque des revendications précédentes, **caractérisé en ce que** le deuxième élément d'appui (376 ; 390) comprend au moins un évidement de passage (346) pour l'élément de couplage (372 ; 380), de sorte que le deuxième élément d'appui (376 ; 390) et l'élément de couplage (372 ; 380) sont mobiles l'un par rapport à l'autre sensiblement parallèlement à la direction de serrage (22).

14. Elément de liaison chirurgical selon l'une quelconque des revendications précédentes, **caractérisé en ce que** le deuxième élément d'appui (376 ; 390) peut être relié, au moyen de l'élément de couplage (372 ; 380) sous l'effet d'un mouvement relatif d'un élément de retenue (350a ; 394 ; 396) reposant sur le deuxième élément d'appui (376 ; 390) par rapport à l'élément de couplage (372 ; 380), à ce dernier.

15. Elément de liaison chirurgical selon l'une quelconque des revendications précédentes, **caractérisé en ce que** l'élément de couplage (372 ; 380) est un corps rigide.

16. Elément de liaison chirurgical selon l'une quelconque des revendications précédentes, **caractérisé en ce que** l'élément de couplage (372 ; 380) est dimensionné de sorte qu'il présente dans une direction transversale par rapport à sa direction longitudinale une largeur supérieure à celle dans la direction transversale perpendiculairement à celle-ci.

17. Elément de liaison chirurgical selon l'une quelconque des revendications précédentes, **caractérisé en ce que** l'élément de couplage (372 ; 380) présente une section transversale sensiblement rectangulaire au moins dans la zone qui peut être positionnée entre des plaques osseuses adjacentes.

18. Elément de liaison chirurgical selon l'une quelconque des revendications précédentes, **caractérisé en ce que** l'élément de retenue (350a ; 394 ; 396) du deuxième élément d'appui (376 ; 390) s'insère transversalement à la direction de serrage (22) dans un évidement de fixation (386).

19. Elément de liaison chirurgical selon l'une quelconque des revendications précédentes, **caractérisé en ce que** l'élément de couplage (372 ; 380) comprend une pluralité d'évidements de fixation (386), lesquels sont agencés dans la direction longitudinale de l'élément de couplage (372 ; 380).

20. Elément de liaison chirurgical selon l'une quelconque des revendications précédentes, **caractérisé en ce que** les évidements de fixation (386) adjacents sont agencés à égale distance les uns des autres.

21. Elément de liaison chirurgical selon l'une quelconque des revendications précédentes, **caractérisé en ce que** l'élément de couplage (372 ; 380) est pourvu d'une baguette d'insertion (384 ; 388) faite d'évidements de fixation.

22. Elément de liaison chirurgical selon la revendication 21, **caractérisé en ce que** la baguette d'insertion (384 ; 388) est réalisée sous la forme d'une baguette encliquetable.

23. Elément de liaison chirurgical selon la revendication 21 ou 22, **caractérisé en ce que** l'élément de couplage (380) comprend au moins deux baguettes d'insertion (384, 388).

24. Elément de liaison chirurgical selon la revendication 23, **caractérisé en ce qu'**au moins deux baguettes d'insertion (384, 388) sont décalées l'une de l'autre en ce qui concerne l'écart des évidements de fixation (386) par rapport au premier élément d'appui (382).

25. Elément de liaison chirurgical selon l'une quelconque des revendications précédentes, **caractérisé en ce qu'**une surface latérale de l'élément de couplage est pourvue d'une baguette d'insertion.

26. Elément de liaison chirurgical selon l'une quelconque des revendications précédentes, **caractérisé en ce qu'**une baguette d'insertion (384 ; 388) est agencée sur l'élément de couplage (372 ; 380) entre les surfaces latérales de l'élément de couplage (372 ; 380).

27. Elément de liaison chirurgical selon l'une quelconque des revendications 22 à 26, **caractérisé en ce qu'**une baguette d'insertion est étagée.

28. Elément de liaison chirurgical selon l'une quelconque des revendications 22 à 27, **caractérisé en ce qu'**une baguette d'insertion est en dents de scie.

29. Elément de liaison chirurgical selon l'une quelconque des revendications précédentes, **caractérisé en ce qu'**une baguette d'insertion (384 ; 388) comprend des évidements rectangulaires (386) comme évidements de fixation.

30. Elément de liaison chirurgical selon l'une quelconque des revendications précédentes, **caractérisé en ce qu'**un évidement de fixation présente une section transversale se rétrécissant.

31. Elément de liaison chirurgical selon l'une quelconque des revendications précédentes, **caractérisé en ce que** le deuxième élément d'appui (376 ; 390) comprend une languette de retenue (394 ; 396), laquelle peut être enfoncée dans un évidement de fixation (386) et au moyen de laquelle le deuxième élément d'appui (376 ; 390) peut être relié à l'élément de couplage (372 ; 380).

32. Elément de liaison chirurgical selon la revendication 31, **caractérisé en ce qu'**une languette de retenue comprend une pluralité de dents de retenue destinées à s'insérer simultanément dans une pluralité d'évidements de fixation.

33. Elément de liaison chirurgical selon la revendication 31 ou 32, **caractérisé en ce qu'**une languette de retenue (350a ; 350b) est agencée en formant un angle avec une surface d'appui (352) du deuxième élément d'appui (344).

34. Elément de liaison chirurgical selon l'une quelconque des revendications précédentes, **caractérisé en ce que** le mouvement relatif entre le deuxième élément d'appui (376 ; 390) et l'élément de couplage (372 ; 380) intervient pour fixer le deuxième élément d'appui (376 ; 390) avec l'élément de couplage (372 ; 380) à partir d'une position non fixée dans une position de fixation.

35. Elément de liaison chirurgical selon l'une quelconque des revendications précédentes, **caractérisé en ce que** des évidements de fixation (386) adjacents sont séparés les uns des autres.

36. Elément de liaison chirurgical selon l'une quelconque des revendications précédentes, **caractérisé en ce que** des évidements de fixation (386) adjacents ne sont pas reliés les uns aux autres.
